# EUROPEAN PATENT APPLICATION

(11) **EP 4 524 887 A1**
(43) Date of publication of application: **19.03.2025**
(21) Application number: 24196923.7
(22) Date of filing: 28.08.2024
(51) Int. Cl.: G06T 7/00

(54) **METHOD FOR DETECTING LESION AND LESION DETECTION PROGRAM**

(30) Priority: 13.09.2023 JP 2023148664
(71) Applicant: FUJITSU LIMITED, Kawasaki-shi, Kanagawa 211-8588 (JP)
(72) Inventor: Baba, Kozo, Kawasaki-shi, Kanagawa, 211-8588 (JP); Takebe, Hiroaki, Kawasaki-shi, Kanagawa, 211-8588 (JP); Miyazaki, Nobuhiro, Kawasaki-shi, Kanagawa, 211-8588 (JP)
(74) Representative: Hoffmann Eitle

(57) **Abstract**

A method for detecting lesion for a computer to execute a process includes a training process that includes calculating a first image feature, based on a plurality of first tomographic image groups obtained by imaging an inside of each of a plurality of first human bodies, for each of the first tomographic image groups, classifying tomographic images included in the plurality of first tomographic image groups into a plurality of second tomographic image groups, according to a range of the first image feature, and generating a plurality of first lesion identification models configured to identify whether or not unit image regions included in the tomographic images to be identified are regions of a particular lesion, for each of the unit image regions, each through machine learning that uses different ones of the plurality of second tomographic image groups from each other, as training data, and a lesion detection process that includes: calculating a second image feature of a same type as the first image feature, based on a plurality of first tomographic images obtained by imaging the inside of a second human body, acquiring, from each of the plurality of first lesion identification models, probabilities of being the regions of the particular lesion, for each of the unit image regions included in the plurality of first tomographic images, by inputting the plurality of first tomographic images to each of the plurality of first lesion identification models, integrating, for each of the unit image regions included in the plurality of first tomographic images, the probabilities acquired from each of the plurality of first lesion identification models, based on the second image feature, to calculate an integrated value, and detecting the regions of the particular lesion from each of the plurality of first tomographic images, based on the integrated value.

## Description

### FIELD

The embodiments discussed herein are related to a method for detecting a lesion and a lesion detection program.

### BACKGROUND

Medical images obtained by computed tomography (CT), magnetic resonance imaging (MRI), and the like are widely used for diagnosis of various diseases. A medical doctor has to interpret a large number of images in diagnostic imaging using medical images, which places a heavy burden on the medical doctor. Therefore, there has been a demand for a technique of supporting diagnostic work of a medical doctor in some way with a computer.

As an example of such a technique, there is a technique for detecting a lesion region from a medical image, using a trained model created through machine learning. For example, an artificial intelligence pipeline for lesion detection and classification including multiple trained machine learning models has been proposed.

### Citation List

### Patent Literature

U.S. Patent Application Publication No. 2022/0270254

### SUMMARY OF INVETION

### TECHNICAL PROBLEM

Incidentally, in a lesion region detection process using a trained model, the detection accuracy can be enhanced as the difference in pixel values (for example, luminance values) between the lesion region and the normal region that does not contain a lesion is larger. However, in a medical image actually captured, there are many cases where a difference in pixel values between the lesion region and the normal region is small, and thus there is a case where it is difficult to enhance the detection accuracy. In addition, in a case where a medical event that can affect the pixel value, such as fat accumulated in an organ, has occurred, the difference in the pixel values between the lesion region and the normal region is sometimes smaller than usual. Furthermore, in some cases, the magnitude relationship in pixel values between the lesion region and the normal region is sometimes reversed.

In one aspect, an object of the embodiments is to provide a method for detecting a lesion and a lesion detection program capable of improving accuracy of lesion region detection from a medical image.

### SOLUTION TO PROBLEM

In an aspect of the embodiments, a method for detecting lesion for a computer to execute a process includes: a training process that includes calculating a first image feature, based on a plurality of first tomographic image groups obtained by imaging an inside of each of a plurality of first human bodies, for each of the first tomographic image groups, classifying tomographic images included in the plurality of first tomographic image groups into a plurality of second tomographic image groups, according to a range of the first image feature, and generating a plurality of first lesion identification models configured to identify whether or not unit image regions included in the tomographic images to be identified are regions of a particular lesion, for each of the unit image regions, each through machine learning that uses different ones of the plurality of second tomographic image groups from each other, as training data, and a lesion detection process that includes calculating a second image feature of a same type as the first image feature, based on a plurality of first tomographic images obtained by imaging the inside of a second human body, acquiring, from each of the plurality of first lesion identification models, probabilities of being the regions of the particular lesion, for each of the unit image regions included in the plurality of first tomographic images, by inputting the plurality of first tomographic images to each of the plurality of first lesion identification models, integrating, for each of the unit image regions included in the plurality of first tomographic images, the probabilities acquired from each of the plurality of first lesion identification models, based on the second image feature, to calculate an integrated value, and detecting the regions of the particular lesion from each of the plurality of first tomographic images, based on the integrated value.

### ADVANTAGEOUS EFFECTS OF INVENTION

In one aspect, the accuracy of lesion region detection from a medical image improves.

### BRIEF DESCRIPTION OF DRAWINGS

FIG. 1 is a diagram illustrating an exemplary configuration and an exemplary process of an information processing device according to a first embodiment;
FIG. 2 is a diagram illustrating an exemplary configuration of a diagnosis support system according to a second embodiment;
FIG. 3 is a diagram illustrating an example of a machine learning model for detecting a lesion region;
FIG. 4 is a diagram illustrating an exemplary configuration of processing functions included in a diagnosis support device;
FIG. 5 is a diagram illustrating an exemplary data configuration of a training dataset;
FIG. 6 is a diagram for explaining a lesion identification model generation process;
FIG. 7 is a diagram for explaining a lesion detection process using lesion identification models;
FIG. 8 is an example of a weight table illustrating a correspondence relationship between image features and weighting factors;
FIG. 9 is an example of a flowchart illustrating a procedure of a model generation process;
FIG. 10 is an example of a flowchart illustrating a procedure of a lesion detection process;
FIG. 11 is a diagram illustrating an exemplary screen display for a result of lesion region detection;
FIG. 12 is a diagram illustrating an exemplary configuration of processing functions included in a diagnosis support device according to a third embodiment;
FIG. 13 is a diagram for explaining a lesion identification model generation process;
FIG. 14 is an example of a weight table illustrating a correspondence relationship between image features and weighting factors;
FIG. 15 is an example of a flowchart illustrating a procedure of a model generation process;
FIG. 16 is an example of a flowchart illustrating a procedure of a lesion detection process;
FIG. 17 is a diagram for explaining a model generation process of a diagnosis support device according to a fourth embodiment;
FIG. 18 is an example of a weight table illustrating a correspondence relationship between image features and weighting factors;
FIG. 19 is an example of a flowchart illustrating a procedure of a model generation process;
FIG. 20 is an example of a flowchart illustrating a procedure of a lesion detection process; and
FIG. 21 is an example of a weight table illustrating a correspondence relationship between image features and weighting factors.

### DESCRIPTION OF EMBODIMENTS

Hereinafter, the embodiments will be described with reference to the drawings.

### [First Embodiment]

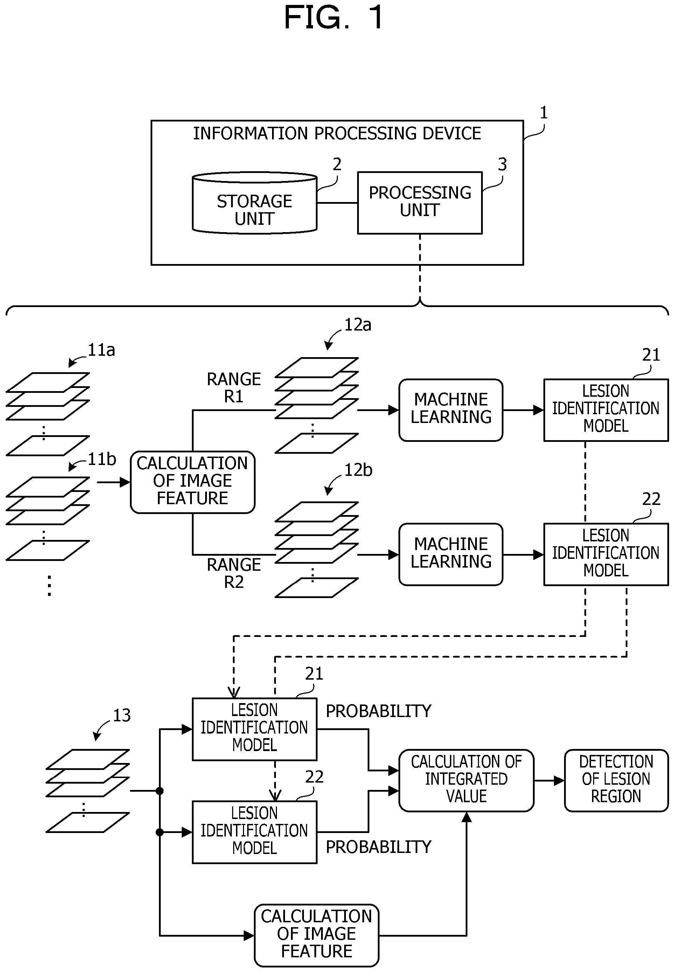

FIG. 1 is a diagram illustrating an exemplary configuration and an exemplary process of an information processing device according to a first embodiment. The information processing device 1 illustrated in FIG. 1 detects a region of a particular lesion from a tomographic image obtained by imaging the inside of a human body. In the following description, as an example, the tomographic image is assumed to be an image captured with a regular interval interposed in a height direction of the human body. For detection of a lesion region, a lesion identification model configured to identify whether or not each unit image region included in an input tomographic image is a lesion region is used. The unit image region refers to, for example, a pixel on the tomographic image or a set (image block) of two or more predetermined number of adjacent pixels. In addition, the information processing device 1 is also enabled to generate such a lesion identification model through machine learning.

This information processing device 1 includes a storage unit 2 and a processing unit 3. The storage unit 2 is a storage area secured in a storage device (not illustrated) included in the information processing device 1. The storage unit 2 stores, for example, a model parameter indicating the lesion identification model. The processing unit 3 is, for example, a processor. In this case, the process of the processing unit 3 as follows is implemented by, for example, the processor executing a predetermined program. The processing unit 3 executes a training process of generating the lesion identification model through machine learning and a lesion detection process of detecting a lesion region using the generated lesion identification model.

In the training process, the process as follows is executed. In this training process, first tomographic image groups 11a, 11b, and so on obtained by imaging the inside of each of a plurality of first human bodies are used as training-purpose data. Based on such first tomographic image groups 11a, 11b, and so on, the processing unit 3 calculates a first image feature for each first tomographic image group. As the first image feature, for example, average luminance in an organ region of each tomographic image included in one set of the first tomographic image group is calculated.

The processing unit 3 classifies the tomographic images included in the first tomographic image groups 11a, 11b, and so on into a plurality of second tomographic image groups according to the range of the first image feature. Since the first image feature is calculated with the first tomographic image group as a unit as described above, the tomographic images are classified in units of the first tomographic image groups in this classification process.

In the example in FIG. 1, the tomographic images included in the first tomographic image groups 11a, 11b, and so on are classified into a second tomographic image group 12a in which the first image feature is included in a range R1 and a second tomographic image group 12b in which the first image feature is included in a range R2. The ranges R1 and R2 are set, for example, by dividing a range that can be taken by the first image feature by a predetermined threshold value. In addition, the ranges R1 and R2 may be set so as to partially overlap each other.

The processing unit 3 generates a plurality of lesion identification models each through machine learning using different ones of the plurality of second tomographic image groups from each other, as training data. In the example in FIG. 1, a lesion identification model 21 is generated through machine learning using the second tomographic image group 12a as training data. In addition, the lesion identification model 22 is generated through machine learning using the second tomographic image group 12b as training data.

After the lesion identification models 21 and 22 are generated, the process as follows is executed in the lesion detection process. The processing unit 3 acquires a third tomographic image group 13 including a plurality of tomographic images obtained by imaging the inside of a second human body. The processing unit 3 calculates a second image feature of the same type as the above-described first image feature, based on the acquired third tomographic image group 13. For example, average luminance in an organ region of each tomographic image included in the third tomographic image group 13 is calculated as the second image feature.

The processing unit 3 inputs each tomographic image included in the third tomographic image group 13 to each of the generated lesion identification models 21 and 22. This causes each of the lesion identification models 21 and 22 to execute a lesion region identification process. The processing unit 3 acquires, from the lesion identification model 21, a probability of being a lesion region, for each unit image region included in the plurality of tomographic images in the third tomographic image group 13. In addition, the processing unit 3 also acquires, from the lesion identification model 22, a probability of being a lesion region, for each unit image region included in the plurality of tomographic images in the third tomographic image group 13.

The processing unit 3 integrates probabilities acquired from each of the lesion identification models 21 and 22 for each of the unit image regions included in the plurality of tomographic images in the third tomographic image group 13, based on the calculated second image feature, to calculate an integrated value. The processing unit 3 detects a lesion region from each tomographic image included in the third tomographic image group 13, based on the calculated integrated value. For example, in a case where the integrated value is equal to or greater than a predetermined threshold value, the corresponding unit image region is identified to be a lesion region, and in a case where the integrated value is less than the threshold value, the corresponding unit image region is identified not to be a lesion region.

Here, in the lesion region detection process using the lesion identification model, in a case where a difference in pixel values (for example, luminance values) between a lesion region and a normal region that does not contain a lesion is small, it becomes difficult to precisely detect a lesion region. In particular, in a case where an anomalous event that can affect a pixel value has occurred, the difference in pixel values between the lesion region and the normal region is sometimes smaller than usual.

As an example of such an anomalous event, there is an event that fat accumulates in an organ such as a liver. In the CT image, the tumor in an organ region appears darker than a normal organ region (normal region). Meanwhile, since the fat region also appears darker, if fat is accumulated in the organ, the entire CT image becomes darker, and the difference in brightness between the lesion region (tumor region) and the normal region becomes small. In some cases, brightness and darkness are reversed between the lesion region and the normal region.

To cope with such a difficulty, in the above-described training process by the processing unit 3, the lesion identification model 21 suitable in a case where the image feature of the input tomographic image is included in the range R1 and the lesion identification model 22 suitable in a case where the image feature of the input tomographic image is included in the range R2 are generated. In a case where an anomalous event of fat accumulation is supposed, one of the lesion identification models 21 and 22 serves as a model suitable in a case where fat is not accumulated, and the other serves as a model suitable in a case where fat is accumulated.

That is, one of the lesion identification models 21 and 22 can accurately detect a lesion region from a tomographic image in which fat is not accumulated, as compared with a lesion identification model generated using training data in which a tomographic image in which fat is not accumulated and a tomographic image in which fat is accumulated are mixed. In addition, the other of the lesion identification models 21 and 22 can accurately detect a lesion region from a tomographic image in which fat is accumulated, as compared with a lesion identification model generated using training data in which a tomographic image in which fat is not accumulated and a tomographic image in which fat is accumulated are mixed.

Then, in the above lesion detection process by the processing unit 3, the probabilities acquired from each of the lesion identification models 21 and 22 are integrated based on the second image feature calculated from the input third tomographic image group 13. In this integration, for example, the processing unit 3 can give a larger weight to a probability from an appropriate lesion identification model according to the second image feature and perform weighted addition of the probabilities.

As an example, assuming that the ranges R1 and R2 do not overlap, the processing unit 3 can increase the weight to be given to a probability from the lesion identification model 21 in a case where the second image feature is included in the range R1. On the other hand, in a case where the second image feature is included in the range R2, the processing unit 3 can increase the weight to be given to a probability from the lesion identification model 22. In a case where one weight to be given to each probability is assumed as zero and the other weight to be given to each probability is assumed as one, the processing unit 3 can also substantially select one of the lesion identification models 21 and 22, based on the second image feature, to execute the lesion detection process.

With such a process, the processing unit 3 may be allowed to accurately detect a lesion region from each tomographic image included in the third tomographic image group 13 regardless of the feature of the images, that is, regardless of the degree of occurrence of an anomalous event regarding the input third tomographic image group 13. Accordingly, the accuracy of lesion region detection from a tomographic image may be improved.

### [Second Embodiment]

Next, a system capable of detecting a lesion region of a liver from a CT image will be described.

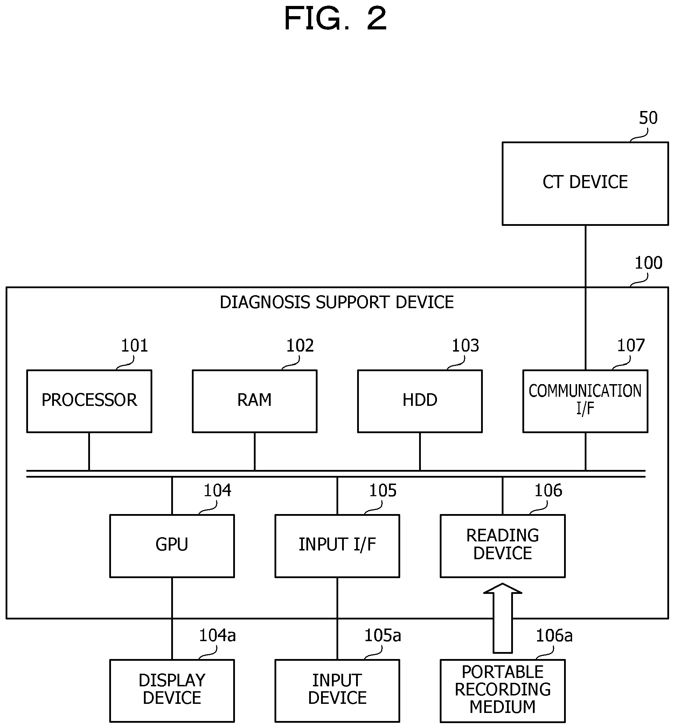

FIG. 2 is a diagram illustrating an exemplary configuration of a diagnosis support system according to a second embodiment. The diagnosis support system illustrated in FIG. 2 is a system that supports an imaging diagnostic work through CT imaging and includes a CT device 50 and a diagnosis support device 100.

The CT device 50 captures an X-ray CT image of a human body. In the present embodiment, the CT device 50 captures a predetermined number of tomographic images in axial planes in an abdominal region including the liver, while changing positions (slice positions) in a height direction of the human body (a direction perpendicular to the axial planes) at predetermined intervals.

The diagnosis support device 100 extracts a region of the liver from each tomographic image captured by the CT device 50 and detects a lesion region, based on image information on the extracted region. In the present embodiment, it is assumed that a tumor (for example, a hemangioma) in the liver is detected as a lesion region. For example, the diagnosis support device 100 displays information indicating a result of lesion region detection on a display device. This ensures that the diagnosis support device 100 supports the imaging diagnostic work of a user (for example, an interpreting doctor).

The diagnosis support device 100 executes detection of a lesion region (or identification of a lesion), using a trained model generated through machine learning. In addition, the diagnosis support device 100 is also enabled to execute a process of generating this trained model through machine learning.

Hereinafter, a hardware configuration of the diagnosis support device 100 will be described with reference to FIG. 2. The diagnosis support device 100 is implemented, for example, as a computer as illustrated in FIG. 2. As illustrated in FIG. 2, the diagnosis support device 100 includes a processor 101, a random access memory (RAM) 102, a hard disk drive (HDD) 103, a graphics processing unit (GPU) 104, an input interface (I/F) 105, a reading device 106, and a communication interface (I/F) 107.

The processor 101 integrally controls the entire diagnosis support device 100. The processor 101 is, for example, a central processing unit (CPU), a micro processing unit (MPU), a digital signal processor (DSP), an application specific integrated circuit (ASIC), or a programmable logic device (PLD). In addition, the processor 101 may be a combination of two or more elements of the CPU, MPU, DSP, ASIC, and PLD. Note that the processor 101 is an example of the processing unit 3 illustrated in FIG. 1.

The RAM 102 is used as a main storage device of the diagnosis support device 100. The RAM 102 temporarily stores at least a part of an operating system (OS) program or an application program to be executed by the processor 101. In addition, the RAM 102 stores various types of data involved in a process by the processor 101.

The HDD 103 is used as an auxiliary storage device of the diagnosis support device 100. The HDD 103 stores the OS program, the application program, and various types of data. Note that another kind of non-volatile storage device such as a solid state drive (SSD) can also be used as the auxiliary storage device.

A display device 104a is coupled to the GPU 104. The GPU 104 displays an image on the display device 104a in accordance with an instruction from the processor 101. Examples of the display device 104a include a liquid crystal display and an organic electroluminescence (EL) display.

An input device 105a is coupled to the input interface 105. The input interface 105 transmits a signal output from the input device 105a to the processor 101. Examples of the input device 105a include a keyboard and a pointing device. Examples of the pointing device include a mouse, a touch panel, a tablet, a touch pad, and a track ball.

A portable recording medium 106a is attached to and detached from the reading device 106. The reading device 106 reads data recorded on the portable recording medium 106a and transmits the read data to the processor 101. Examples of the portable recording medium 106a include an optical disc and a semiconductor memory.

The communication interface 107 transmits and receives data to and from another device such as the CT device 50 via a network.

The processing functions of the diagnosis support device 100 can be implemented with the hardware configuration described above.

Incidentally, as described above, the lesion region detection process from a medical image can be executed using, for example, a trained model generated through machine learning.

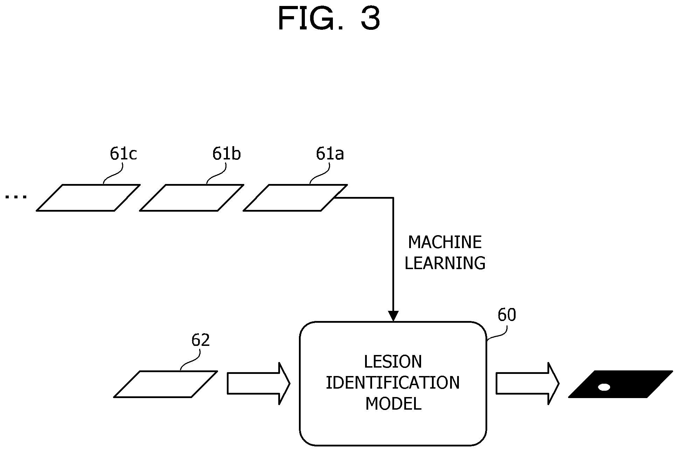

FIG. 3 is a diagram illustrating an example of a machine learning model for detecting a lesion region. A lesion identification model 60 illustrated in FIG. 3 is a trained model that identifies whether or not each pixel of a tomographic image 62 is a particular lesion region when the tomographic image 62 is input. Here, it is assumed that the tomographic image 62 is a tomographic image in an axial plane. In practice, the lesion identification model 60 calculates a probability of being a lesion region for each pixel of the input tomographic image 62 and identifies a pixel whose probability is equal to or greater than a predetermined value, as a lesion region.

This lesion identification model 60 is generated through machine learning (for example, deep learning) similarly using tomographic images 61a, 61b, 61c, and so on in axial planes as teacher data. A label indicating whether or not a pixel is a lesion region for each pixel is appended to these tomographic images 61a, 61b, 61c, and so on, and these labels are used as ground truth data at the time of machine learning.

The diagnosis support device 100 of the present embodiment can detect a lesion region, using such the lesion identification model 60. In addition, as another example of the lesion identification model, there is also one that identifies whether or not a partial image (patch image) of a certain size treated as a unit is a lesion region. In this case, each tomographic image used as training data is divided into patch images of a certain size, and a label indicating whether or not a divided patch image is a lesion region for each of the divided patch images is appended to the patch images. Then, machine learning in units of patch images is performed using each patch image as teacher data and using the label as ground truth data, whereby a lesion identification model is generated. At the time of inference, the tomographic image input to the lesion identification model is divided into patch images of a certain size, and the lesion identification model identifies whether or not each patch image is a lesion region.

Incidentally, in the lesion identification process using the lesion identification model as described above, it is considered that the larger the luminance difference in the organ region on the CT image between the lesion region and the normal region that does not contain a lesion, the higher the accuracy of lesion region identification. In the real medical practice, the CT image is captured using a contrast medium in order to emphasize the luminance difference between the lesion region and the normal region. However, there is a case where side effects occur in a person subject to diagnosis due to the use of the contrast medium. Therefore, the person subject to diagnosis to whom the contrast medium is administrable is limited to a person subject to diagnosis in a relatively good state such as a stage where there is no subjective symptom due to the disease or a stage where the disease is suspected.

For such reasons, it is desired to be able to accurately identify a lesion region from a CT image captured without using a contrast medium (herein expressed as a "simple CT image"). In addition, the simple CT image is often captured at a stage where no particular disease is suspected, such as a medical examination. Enabling detection of a serious disease such as cancer from the CT image captured at such a stage leads to finding a disease early in a true sense. Furthermore, if a lesion region can be automatically and accurately identified from the simple CT image, it becomes possible to find a disease even in a medical institution where a medical specialist of diagnostic imaging is not resident.

However, in the simple CT image, the luminance difference between the lesion region and the normal region in the organ region is small, and there is a case where the lesion region may not be accurately identified. For example, in a case where a tumor is detected from the liver, since the luminance value of the tumor region is lower than that of the normal region inside the liver in the simple CT image, the tumor region appears darker. However, in a case where fat is accumulated in the liver, fat also appears dark on the simple CT image, and thus the luminance value of the entire liver region decreases. As a result, the luminance difference between the tumor region and the normal region becomes smaller, and it becomes difficult to identify the tumor region with high accuracy. In some cases, the magnitude relationship in luminance between the tumor region and the normal region is reversed, and it becomes more difficult to identify the tumor region with high accuracy.

In view of such a point, in a case where a simple CT image including fat and a simple CT image not including fat are mixed in simple CT images used as training data at the time of machine learning, it is considered that it is difficult to generate a lesion identification model with high identification accuracy. This is because the machine learning will be performed using the simple CT images with varying luminance differences between the lesion region and the normal region or the simple CT images having different magnitude relationships in luminance between the lesion region and the normal region.

Thus, in the present embodiment, a CT image group made up of the simple CT images used as training data is classified into a plurality of CT image groups each having a different degree of occurrence of an anomalous event that can change the relationship in luminance between the lesion region and the normal region. In the above example, fat accumulation is assumed as an anomalous event. Such classification into the CT image groups can be simply performed based on findings by the interpreting doctor.

The diagnosis support device 100 according to the present embodiment generates a plurality of lesion identification models, using every one of different CT image groups from each other among the plurality of CT image groups classified in this manner, as training data. This generates a plurality of lesion identification models according to variations in the feature of the input CT images.

At the time of inference, the diagnosis support device 100 acquires a CT image group made up of the simple CT images and estimates the degree of occurrence of an anomalous event in the acquired CT image group from the image feature of the CT image group. At the same time, the diagnosis support device 100 executes the lesion identification process on the CT image group, using each of the plurality of lesion identification models described above, and acquires probability values as to being a lesion region from each lesion identification model for each pixel of the CT images included in the CT image group. The diagnosis support device 100 integrates the acquired probability values at a ratio according to the image feature to output a final identification result.

In the integration of the probability values, the ratio of the probability values from the lesion identification model suitable for the degree of occurrence of an anomalous event in the CT image group can be raised based on the image feature, and the final identification result can be determined. It is also possible to select the probability values from one lesion identification model optimal for the degree of occurrence of an anomalous event in the CT image group to determine a final identification result. Therefore, the lesion identification accuracy from the simple CT image may be improved.

In the following description, a case will be mainly exemplified in which a lesion identification model generated using a CT image with an anomalous finding in which fat is accumulated and a lesion identification model generated using a CT image with a normal finding in which fat is not accumulated are used.

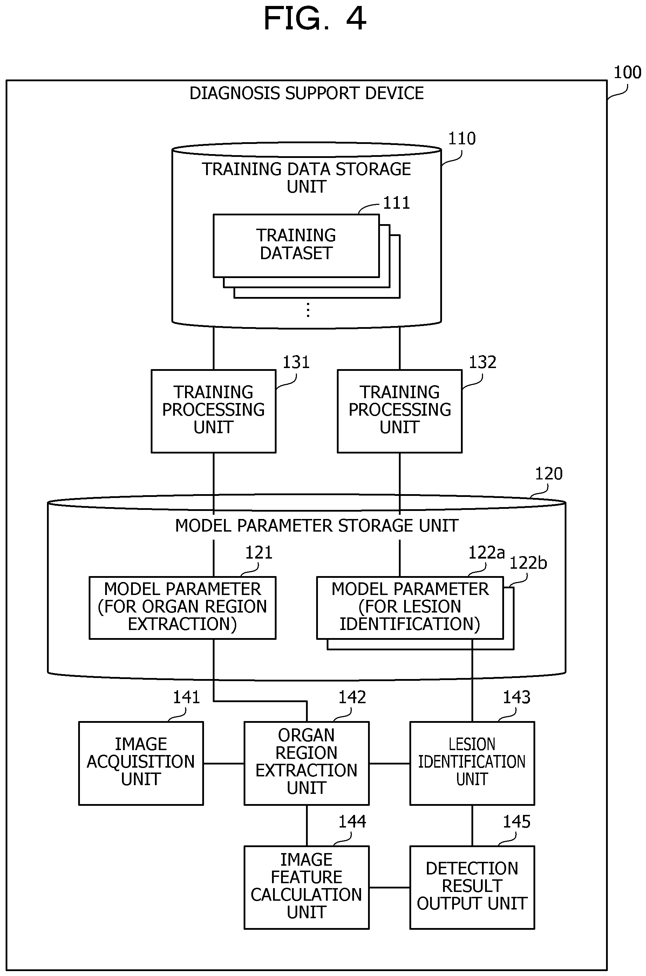

FIG. 4 is a diagram illustrating an exemplary configuration of processing functions included in the diagnosis support device. The diagnosis support device 100 includes a training data storage unit 110, a model parameter storage unit 120, training processing units 131 and 132, an image acquisition unit 141, an organ region extraction unit 142, a lesion identification unit 143, an image feature calculation unit 144, and a detection result output unit 145.

The training data storage unit 110 and the model parameter storage unit 120 are storage areas secured in a storage device included in the diagnosis support device 100, such as the RAM 102 and the HDD 103.

The training data storage unit 110 stores a plurality of sets of training datasets 111 used to generate a lesion identification model through machine learning. The training dataset 111 includes a CT image (tomographic image) set for one imaging subject person. In addition, as the training datasets 111, there are a dataset including a CT image set with normal findings and a dataset including a CT image set with anomalous findings (findings that fat is present). In the diagnosis support device 100, two lesion identification models according to variations in findings are generated using these training datasets 111. Note that every CT image included in each training dataset 111 is a simple CT image captured without using a contrast medium.

The model parameter storage unit 120 stores a model parameter 121 corresponding to an organ region extraction model that is a trained model for organ region (liver region) extraction, and model parameters 122a and 122b separately corresponding to the above two lesion identification models. Note that the model parameters stored in the model parameter storage unit 120 are data for forming a trained model and include, for example, a weighting factor between nodes on a neural network.

The processes of the training processing units 131 and 132, the image acquisition unit 141, the organ region extraction unit 142, the lesion identification unit 143, the image feature calculation unit 144, and the detection result output unit 145 are implemented by, for example, the processor 101 executing an application program.

The training processing units 131 and 132 operate in a training phase in which the organ region extraction model and the lesion identification models are generated through machine learning.

The training processing unit 131 generates the organ region extraction model by performing machine learning using each training dataset 111 stored in the training data storage unit 110. The training processing unit 131 stores the model parameter 121 indicating the generated organ region extraction model, in the model parameter storage unit 120.

The training processing unit 132 generates the lesion identification models by performing machine learning using each training dataset 111 stored in the training data storage unit 110. At this time, the training processing unit 132 generates the lesion identification model corresponding to normal findings, using the training dataset 111 including the CT image set with normal findings (training dataset with normal findings). At the same time, the training processing unit 132 generates the lesion identification model corresponding to anomalous findings, using the training dataset 111 including the CT image set with anomalous findings (training dataset with anomalous findings). The training processing unit 132 stores the model parameters 122a and 122b separately corresponding to the generated two lesion identification models, in the model parameter storage unit 120.

The image acquisition unit 141, the organ region extraction unit 142, the lesion identification unit 143, the image feature calculation unit 144, and the detection result output unit 145 operate in an inference phase in which a lesion region is detected using the generated organ region extraction model and lesion identification models.

The image acquisition unit 141 acquires a CT image set obtained by imaging a person subject to diagnosis, from the CT device 50. The acquired CT image set is an image set of simple CT images captured without using a contrast medium.

The organ region extraction unit 142 extracts a CT image including the organ region (liver region) from the CT image set, using the organ region extraction model based on the model parameter 121.

The lesion identification unit 143 executes the lesion identification process, using the lesion identification model based on the model parameter 122a, for each CT image including the organ region, and outputs probability values as to being a lesion region, for each pixel. In addition, the lesion identification unit 143 executes the lesion identification process, using the lesion identification model based on the model parameter 122b, for each CT image including the organ region, and outputs probability values as to being a lesion region, for each pixel.

The image feature calculation unit 144 calculates the image feature from the organ region in the image region of the CT image included in the CT image set. In the present embodiment, average luminance in the organ region is calculated as the image feature.

The detection result output unit 145 integrates lesion identification results by the two lesion identification models and outputs a final result of lesion region detection. Specifically, for each pixel of the CT image including the organ region, the detection result output unit 145 performs weighted addition on the probability values calculated by the two lesion identification models, using a weighting factor according to a result of image feature calculation. The detection result output unit 145 compares the calculated value of the weighted addition with a predetermined threshold value and identifies a pixel whose calculated value is equal to or greater than the threshold value, as a pixel of the lesion region.

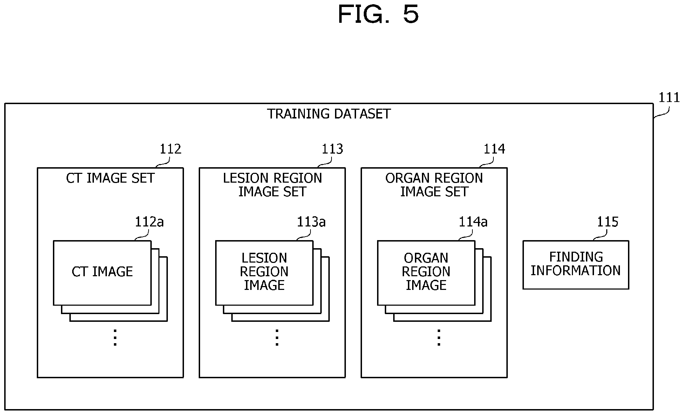

FIG. 5 is a diagram illustrating an exemplary data configuration of the training dataset. The training dataset 111 stored in the training data storage unit 110 includes a CT image set 112, a lesion region image set 113, an organ region image set 114, and finding information 115.

The CT image set 112 includes a plurality of CT images 112a (tomographic images) obtained by one time of imaging for one imaging subject person.

The lesion region image set 113 includes lesion region images 113a corresponding to each of the plurality of CT images 112a included in the CT image set 112. The lesion region image 113a is an image in which a label indicating whether or not a pixel of the corresponding CT image 112a is a lesion region is associated with each pixel. Each label of the lesion region image 113a is used as a ground truth label when the lesion identification models are generated through machine learning.

The organ region image set 114 includes organ region images 114a corresponding to each of the plurality of CT images 112a included in the CT image set 112. The organ region image 114a is an image in which a label indicating whether or not a pixel of the corresponding CT image 112a is an organ region (liver region) is associated with each pixel. Each label of the organ region image 114a is used as a ground truth label when the organ region extraction model is generated through machine learning.

The finding information 115 is information indicating whether the plurality of CT images 112a included in the CT image set 112 has a normal finding or an anomalous finding. The finding information 115 is generated with the CT image set 112 as a unit. For example, in a case where the interpreting doctor judges that fat is accumulated inside the organ to some extent or more when comprehensively viewing the CT images 112a included in the CT image set 112, these CT images 112a are treated to have anomalous findings, and otherwise, these CT images 112a are treated to have normal findings. In this manner, the finding information 115 is information generated based on the medical finding by the interpreting doctor.

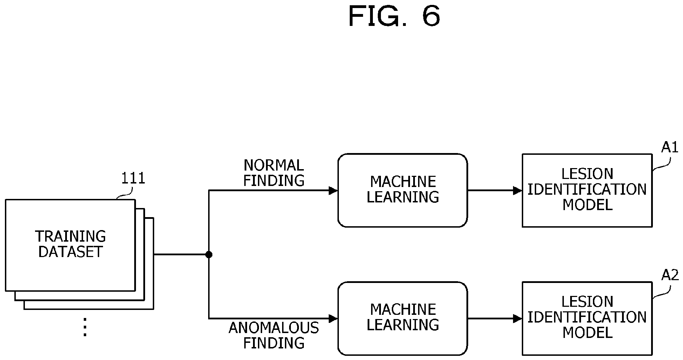

FIG. 6 is a diagram for explaining a lesion identification model generation process. The training dataset 111 stored in the training data storage unit 110 is classified into the training dataset 111 including the CT image set 112 with normal findings (the training dataset 111 with normal findings) and the training dataset 111 including the CT image set 112 with anomalous findings (the training dataset 111 with anomalous findings), based on the finding information 115.

The training processing unit 132 extracts the training dataset 111 with normal findings, based on the finding information 115, from the training dataset 111 stored in the training data storage unit 110. The training processing unit 132 performs machine learning, using each CT image 112a and the lesion region image 113a included in the extracted training dataset 111, thereby generating a lesion identification model A1 corresponding to normal findings. In practice, the CT image 112a including the organ region among the CT images 112a included in the CT image set 112 with normal findings and the lesion region image 113a corresponding this CT image 112a are used for machine learning. This ensures that the model parameter 122a indicating the lesion identification model A1 is stored in the model parameter storage unit 120.

In addition, the training processing unit 132 extracts the training dataset 111 with anomalous findings, based on the finding information 115, from the training dataset 111 stored in the training data storage unit 110. The training processing unit 132 performs machine learning, using each CT image 112a and the lesion region image 113a included in the extracted training dataset 111, thereby generating a lesion identification model A2 corresponding to anomalous findings. In practice, the CT image 112a including the organ region among the CT images 112a included in the CT image set 112 with anomalous findings and the lesion region image 113a corresponding this CT image 112a are used for machine learning. This ensures that the model parameter 122b indicating the lesion identification model A2 is stored in the model parameter storage unit 120.

Through the above model generation process, the lesion identification model A1 suitable for lesion detection from the CT image group in which fat is not accumulated in the organ region and the lesion identification model A2 suitable for lesion detection from the CT image group including fat in the organ region are individually generated.

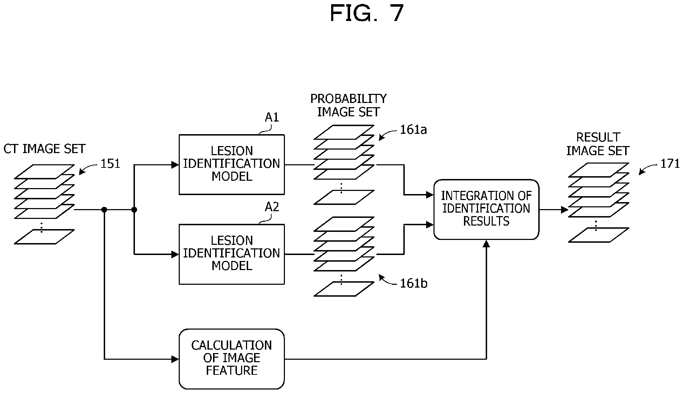

FIG. 7 is a diagram for explaining the lesion detection process using lesion identification models.

A person subject to diagnosis is imaged by the CT device 50, and a CT image set 151 is obtained. A CT image including the organ region is extracted from this CT image set 151, using the organ region extraction model, and each extracted CT image is input to both of the lesion identification models A1 and A2.

The lesion identification model A1 executes the lesion identification process on each input CT image in units of pixels and outputs probability values indicating a probability of being a lesion region, for each pixel. This generates, for example, a probability image set 161a including probability images corresponding to each of the input CT images. The probability image included in the probability image set 161a is an image in which a probability value is associated with each pixel of the corresponding CT image.

In addition, the lesion identification model A2 executes the lesion identification process on each input CT image in units of pixels and outputs probability values indicating a probability of being a lesion region, for each pixel. This generates, for example, a probability image set 161b including probability images corresponding to each of the input CT images. The probability image included in the probability image set 161b is an image in which a probability value is associated with each pixel of the corresponding CT image.

Note that, for example, in a case where the lesion identification models A1 and A2 are machine learning models using a neural network, the above probability value is output from a last layer (output layer) of the neural network.

Furthermore, each CT image including the organ region is also input to the image feature calculation unit 144. The image feature calculation unit 144 extracts a luminance value of each pixel of the organ region in the image region of each input CT image and calculates an average value of the extracted luminance values (average luminance), as an image feature.

The detection result output unit 145 integrates the respective lesion identification results by the lesion identification models A1 and A2 and outputs a final result of lesion region detection. At this time, the detection result output unit 145 integrates the probability values in the probability images in the probability image set 161a and the probability values in the probability images in the probability image set 161b at a ratio according to the calculated image feature (average luminance) for each pixel of the CT image treated as an object to be processed. In this integration process, weighted addition using a weighting factor according to the image feature is performed on the probability value of each probability image.

The detection result output unit 145 compares the integrated probability values with a predetermined threshold value and outputs a final detection result indicating whether or not each pixel of the CT image is a lesion region. For example, a result image is generated in which information indicating whether or not a pixel of the CT image treated as an object to be processed is a lesion region is associated with each pixel. A result image set 171 illustrated in FIG. 7 includes result images corresponding to each of a plurality of CT images treated as an object to be processed.

According to the lesion detection process described above, the diagnosis support device 100 can estimate the degree of accumulation of fat in the CT image group, depending on the image feature calculated from the input CT image group. For example, it is estimated that more fat is accumulated as the average luminance is lower. Then, the diagnosis support device 100 may appropriately integrate the lesion identification results by each of the lesion identification models A1 and A2 according to the degree of accumulation of fat estimated based on the image feature and obtain a final lesion identification result.

Accordingly, the lesion region may be accurately detected from the simple CT image. For example, even in a case where the luminance difference between the lesion region and the normal region in the organ region is small, or a case where the magnitude relationship in luminance between the lesion region and the normal region is not uniform, or a case where the luminance in the lesion region is not uniform, the accuracy of lesion region detection may be improved.

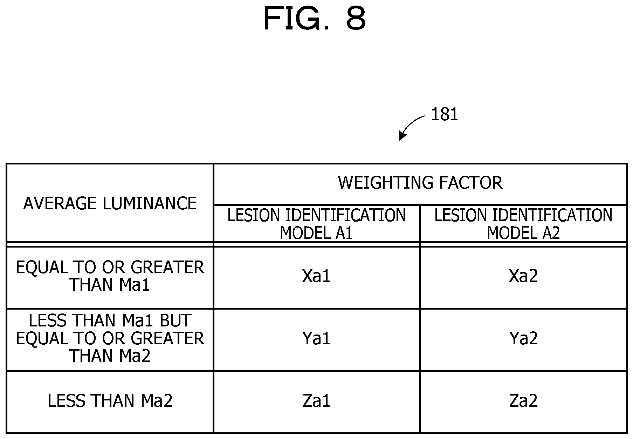

FIG. 8 is an example of a weight table illustrating a correspondence relationship between image features and weighting factors. The detection result output unit 145 can integrate the respective identification results of the lesion identification models A1 and A2 with reference to, for example, a weight table 181 as illustrated in FIG. 8.

In FIG. 8, as an example, a region that can be taken by the calculated average luminance is divided into three regions, using threshold values Ma1 and Ma2 (where Ma1 > Ma2 holds), and weighting factors for each of the lesion identification models A1 and A2 are associated with each of the divided regions. In a case where the average luminance is equal to or greater than the threshold value Ma1, Xa1 and Xa2 are applied as weighting factors corresponding to the lesion identification models A1 and A2, respectively. In a case where the average luminance is less than the threshold value Ma1 but equal to or greater than the threshold value Ma2, Ya1 and Ya2 are applied as weighting factors corresponding to the lesion identification models A1 and A2, respectively. In a case where the average luminance is less than the threshold value Ma2, Za1 and Za2 are applied as weighting factors corresponding to the lesion identification models A1 and A2, respectively.

Here, the weighting factors Xa1, Xa2, Ya1, Ya2, Za1, and Za2 each take a value equal to or greater than zero but equal to or less than one, and Xa1 + Xa2 = Ya1 + Ya2 = Za1 + Za2 = 1 holds. In a case where a weighting factor corresponding to one of the lesion identification models A1 and A2 has one, a weighting factor corresponding to the other has zero. In this case, the probability values output from the one lesion identification model will be used as it is.

In addition, it is estimated that more fat is accumulated as the average luminance is lower. Therefore, as the average luminance is lower, the weighting factors for the lesion identification model A2 corresponding to the anomalous findings are set to higher values. Specifically, Xa1 > Ya1 > Za1 and Xa2 < Ya2 < Za2 are satisfied. In addition, Xa1 > Xa2 and Za1 < Za2 are satisfied. The magnitude relationship between Ya1 and Ya2 only has to be determined according to the setting values of the threshold values Ma1 and Ma2.

By using such weighting factors, the detection result output unit 145 can increase the weight for the probability values by a lesion identification model having the condition of the CT images used as the training data closer to the condition of the input CT image group, among the lesion identification models A1 and A2, and can obtain the final lesion identification result. As a result, a lesion region may be accurately detected from the simple CT image.

Note that, as the image feature, for example, a percentage of high luminance pixels having luminance equal to or greater than a predetermined threshold value in the organ region in the CT image group corresponding to one person, as well as the average luminance of the organ region as described above, can also be used. Such a percentage of high luminance pixels is calculated as a percentage of the number of high luminance pixels included in the organ region to the total number of pixels of the organ region in the CT image group. In a case where such a percentage of high luminance pixels is used as the image feature, the weighting factors for the lesion identification model A2 corresponding to anomalous findings only have to be set to higher values as the percentage of high luminance pixels is lower.

In addition, FIG. 6 illustrates an example in which two lesion identification models A1 and A2 are generated using two CT image sets having different findings. However, as another example, three or more lesion identification models may be generated using three or more CT image sets with different findings. For example, as the lesion identification model corresponding to anomalous findings, a lesion identification model based on a CT image set in which the degree of accumulation of fat is mild and a lesion identification model based on a CT image set in which the degree of accumulation of fat is severe may be generated. In this case, a lesion identification model corresponding to normal findings and two lesion identification models corresponding to anomalous findings are used at the time of lesion detection. In a case where three or more lesion identification models are used in this manner, at the time of lesion detection, the lesion identification results by each of the three or more lesion identification models can be simply integrated at a ratio according to the image feature.

In addition, as an anomalous event that can change the relationship in luminance between the lesion region and the normal region, for example, calcification in the liver is also considered apart from the accumulation of fat as described above. The liver region in which calcification has developed appears brightly on the CT image. For this reason, in a case where the calcification has developed, the luminance value of the entire liver region rises. As a result, the luminance difference between the lesion region and the normal region becomes smaller, and it becomes difficult to identify the lesion region with high accuracy.

From this fact, for example, the diagnosis support device 100 may use a CT image set in which calcification is not produced, to generate a lesion identification model corresponding to normal findings, and use a CT image set in which calcification is produced, to generate a lesion identification model corresponding to anomalous findings. In this case, at the time of lesion identification, for example, the percentage of the high luminance pixels described above can be used as the image feature. That is, the lesion identification results (probability values) from each of the lesion identification models are integrated at a ratio according to the percentage of high luminance pixels in the input CT image set. At this time, the weighting factors corresponding to the lesion identification model corresponding to anomalous findings only have to be set to higher values as the percentage of the high luminance pixels increases.

In addition, in the above description, the plurality of lesion identification models has been generated in consideration of the degree of occurrence of one type of anomalous event that can change the relationship in luminance between the lesion region and the normal region. However, as another example, a plurality of lesion identification models may be generated in consideration of the degrees of occurrence of a plurality of types of anomalous events that can change the relationship in luminance between the lesion region and the normal region.

For example, both of fat accumulation and calcification may be considered as anomalous events. In this case, for example, the lesion identification model corresponding to normal findings is generated using a CT image set in which neither fat accumulation nor calcification is observed. In addition, as the lesion identification model corresponding to the anomalous findings, a lesion identification model based on a CT image set in which accumulation of fat is observed and a lesion identification model based on a CT image set in which calcification is observed are generated. At the time of lesion detection, the lesion identification results by the respective lesion identification models only have to be integrated at a ratio according to the image feature.

Next, processes of the diagnosis support device 100 according to the second embodiment will be described with reference to flowcharts.

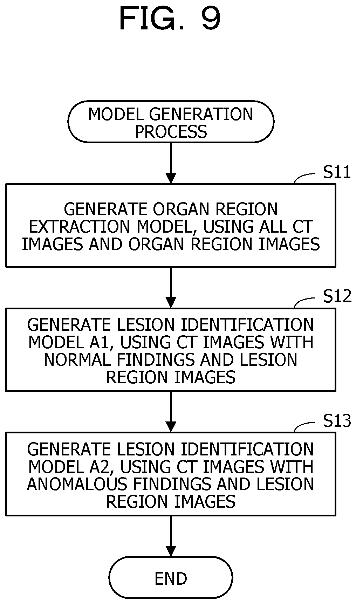

First, a procedure of the model generation process for generating the lesion identification models A1 and A2 described above will be described with reference to FIG. 9. FIG. 9 is an example of a flowchart illustrating a procedure of the model generation process.

[Step S11] The training processing unit 131 generates the organ region extraction model by performing machine learning using all the CT images 112a stored in the training data storage unit 110 and the organ region images 114a corresponding to each of the CT images 112a. In this machine learning, information on each pixel of the organ region images 114a is used as ground truth data. The training processing unit 131 stores the model parameter 121 indicating the generated organ region extraction model, in the model parameter storage unit 120.

Note that, in the present embodiment, as an example, the organ region extraction model is generated using the same training-purpose data as at the time of generating the lesion identification models A1 and A2. However, as the organ region extraction model, an organ region extraction model generated in advance using other training-purpose data may be used.

[Step S12] The training processing unit 132 extracts the training dataset 111 with normal findings, based on the finding information 115, from the training dataset 111 stored in the training data storage unit 110. Furthermore, the training processing unit 132 extracts the CT image 112a including the organ region, from the extracted training dataset 111, based on the organ region image 114a.

The training processing unit 132 performs machine learning, using the extracted CT images 112a and the lesion region images 113a corresponding to these CT image 112a, thereby generating the lesion identification model A1 corresponding to normal findings. In this machine learning, information on each pixel of the lesion region images 113a is used as ground truth data. The training processing unit 132 stores the model parameter 122a indicating the generated lesion identification model A1, in the model parameter storage unit 120.

[Step S13] The training processing unit 132 extracts the training dataset 111 with anomalous findings, based on the finding information 115, from the training dataset 111 stored in the training data storage unit 110. Furthermore, the training processing unit 132 extracts the CT image 112a including the organ region, from the extracted training dataset 111, based on the organ region image 114a.

The training processing unit 132 performs machine learning, using the extracted CT images 112a and the lesion region images 113a corresponding to these CT image 112a, thereby generating the lesion identification model A2 corresponding to anomalous findings. In this machine learning, information on each pixel of the lesion region images 113a is used as ground truth data. The training processing unit 132 stores the model parameter 122b indicating the generated lesion identification model A2, in the model parameter storage unit 120.

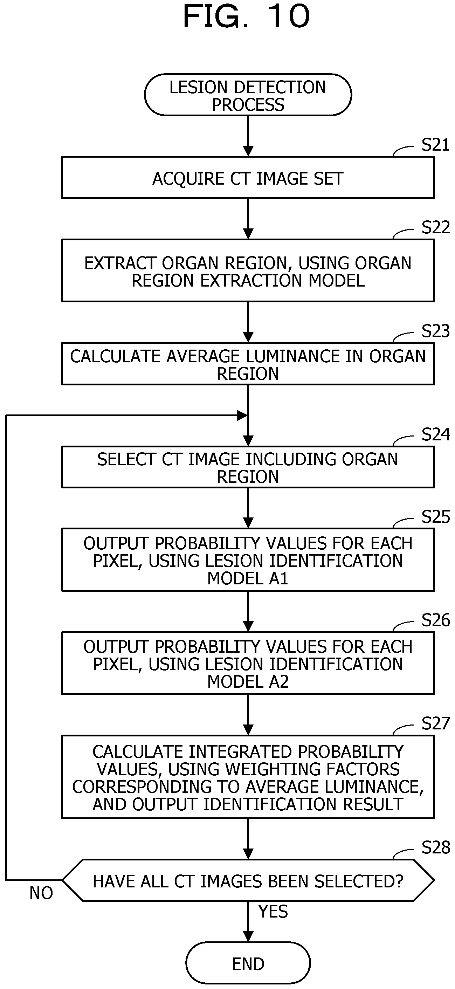

Next, a procedure of the lesion detection process for detecting a lesion region using the generated lesion identification models A1 and A2 will be described with reference to FIG. 10. FIG. 10 is an example of a flowchart illustrating a procedure of the lesion detection process.

[Step S21] The image acquisition unit 141 acquires a CT image set obtained by imaging a person subject to diagnosis, from the CT device 50.

[Step S22] The organ region extraction unit 142 inputs each CT image included in the CT image set to the organ region extraction model based on the model parameter 121. This prompts the organ region extraction unit 142 to identify whether or not each pixel of each CT image is an organ region. For example, an organ region image in which information indicating whether or not a pixel of the CT image is an organ region is associated with each pixel is generated.

The organ region extraction unit 142 extracts a CT image including the organ region (a CT image including one or more pixels of the organ region pixels) from the CT image set. In next step S23 and the subsequent steps, processes are executed using the extracted CT images.

[Step S23] The image feature calculation unit 144 acquires the pixel value of each pixel of the organ region from all the CT images including the organ region and converts the acquired pixel value into the luminance value. The image feature calculation unit 144 calculates an average value of the luminance values after conversion, that is, average luminance in the organ region.

[Step S24] The lesion identification unit 143 selects the CT image to be processed, from among the CT images including the organ region.

[Step S25] The lesion identification unit 143 inputs the CT image to be processed to the lesion identification model A1 based on the model parameter 122a. This prompts the lesion identification unit 143 to output probability values as to being a lesion region, for each pixel of the CT image.

[Step S26] The lesion identification unit 143 inputs the CT image to be processed to the lesion identification model A2 based on the model parameter 122b. This prompts the lesion identification unit 143 to output probability values as to being a lesion region, for each pixel of the CT image.

[Step S27] The detection result output unit 145 acquires the weighting factors corresponding to the average luminance calculated in step S23 from the weight table 181. This ensures that the weighting factors corresponding to each of the lesion identification models A1 and A2 are acquired. The detection result output unit 145 multiplies each of the probability values output in steps S25 and S26 by the weighting factors corresponding to the lesion identification models A1 and A2 and adds up each of the obtained multiplication results, thereby calculating integrated probability values for each pixel. The detection result output unit 145 compares the calculated integrated probability value with a predetermined threshold value, identifies a pixel having an integrated probability value equal to or greater than the threshold value, as a lesion region, and identifies a pixel having an integrated probability value less than the threshold value, as a normal region. For example, the detection result output unit 145 generates and outputs a result image in which information indicating whether or not a pixel of the CT image is a lesion region is associated with each pixel.

[Step S28] The lesion identification unit 143 verifies whether all the CT images including the organ region have been selected. In a case where there is an unselected CT image, the process proceeds to step S24, and one unselected CT image is selected. On the other hand, in a case where all the applicable CT images have been selected, the lesion detection process ends.

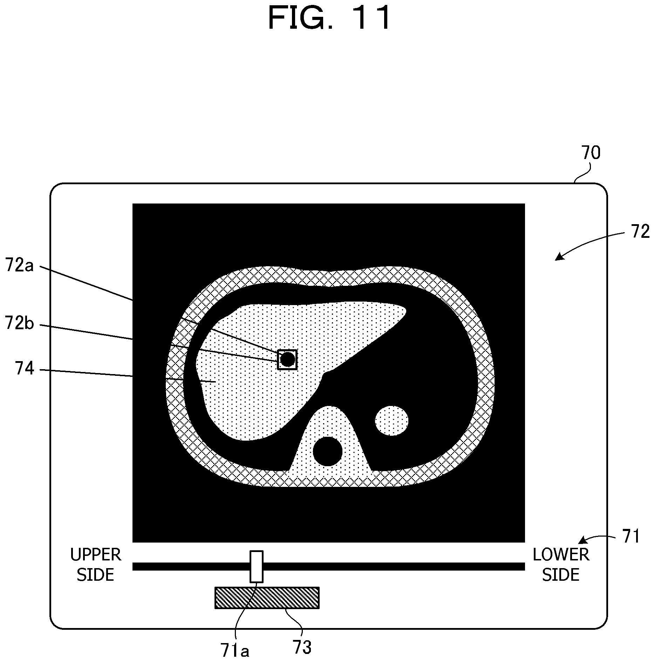

FIG. 11 is a diagram illustrating an exemplary screen display for a result of lesion region detection. The detection result output unit 145 can output information indicating the result of lesion region detection, using the result image generated for each CT image including the organ region. For example, the detection result output unit 145 can display a result display screen 70 as illustrated in FIG. 11 on the display device 104a. The result display screen 70 includes a slice selection portion 71, a CT image display portion 72, and a lesion-detected region display portion 73.

In the slice selection portion 71, a slice plane of the CT image to be displayed in the CT image display portion 72 can be selected by moving a handle 71a on a slider. The CT image corresponding to the slice plane selected in the slice selection portion 71 is displayed in the CT image display portion 72. In this CT image, a lesion region is displayed based on the result image corresponding to the same slice plane. In FIG. 11, as an example, a rectangle 72b surrounding a lesion region 72a is displayed in a superimposed manner in the CT image. In addition, as another display method, the lesion region 72a may be displayed in a particular color. This lesion region 72a is a region of a pixel associated with information indicating a lesion region in the result image. Furthermore, an organ region 74 may be displayed in another particular color.

The lesion-detected region display portion 73 indicates a range of slice planes in which a lesion region is detected (slice planes corresponding to result images in which information indicating a lesion region is included in one or more pixels) in a movable region of the handle 71a in the slice selection portion 71. With this display of the lesion-detected region display portion 73, a user may be allowed to easily recognize the slice planes in which a lesion region is detected, to quickly display the tomographic images corresponding to these slice planes, and to confirm the lesion region in the displayed tomographic images.

### [Third Embodiment]

Next, an example in which a part of the processes of the diagnosis support device 100 according to the second embodiment is modified will be described as a third embodiment. The third embodiment is different from the second embodiment in the following points. In the third embodiment, the CT image set for training is classified into a plurality of CT image groups according to the range of the image feature calculated from the CT image set. Then, a plurality of lesion identification models is generated using each of the classified CT image groups as training data.

In the following description, as an example, it is assumed that a CT image set for training is classified into two CT image groups and two lesion identification models are generated.

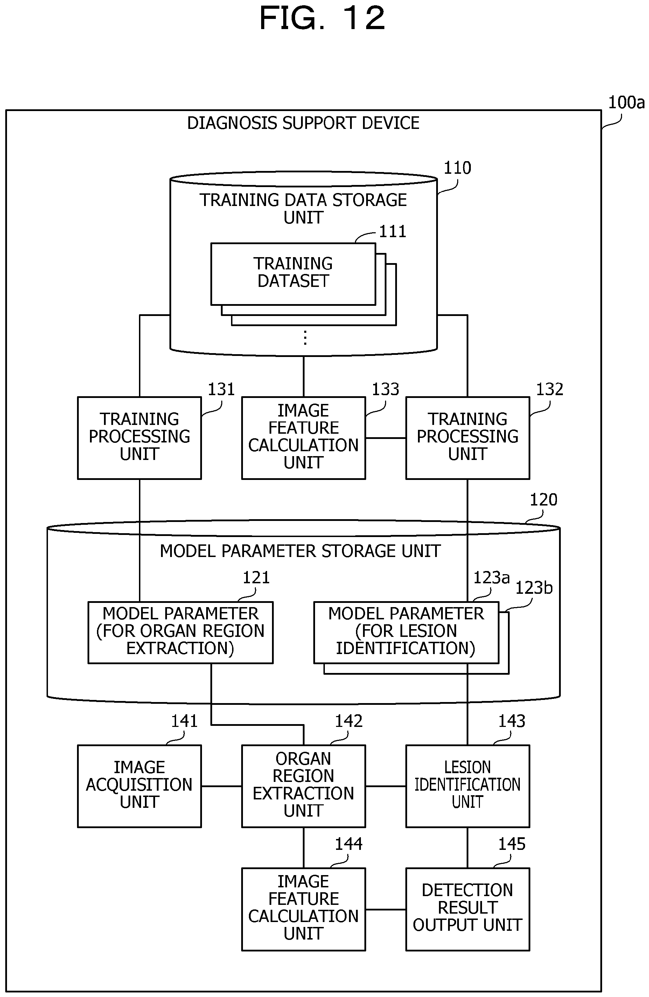

FIG. 12 is a diagram illustrating an exemplary configuration of processing functions included in a diagnosis support device according to the third embodiment. In FIG. 12, the same constituent elements as in FIG. 4 are indicated with the same reference signs denoted.

The diagnosis support device 100a according to the third embodiment further includes an image feature calculation unit 133 in addition to training processing units 131 and 132, an image acquisition unit 141, an organ region extraction unit 142, a lesion identification unit 143, an image feature calculation unit 144, and a detection result output unit 145 illustrated in FIG. 4. The process of the image feature calculation unit 133 is implemented by a processor included in the diagnosis support device 100a executing a predetermined program. The image feature calculation unit 133 calculates an image feature of the same type as the image feature calculation unit 144 from each of CT image sets 112 stored in a training data storage unit 110.

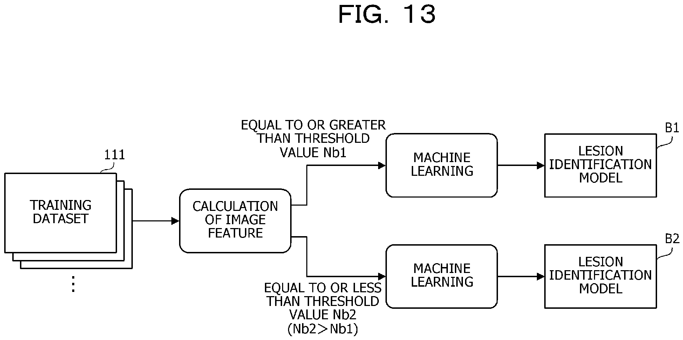

In addition, the CT image set 112 stored in the training data storage unit 110 is classified into a CT image group in which the calculated image feature is included in a first range and a CT image group in which the calculated image feature is included in a second range. The training processing unit 132 generates a lesion identification model B1 (see FIG. 13) through machine learning using CT images included in one CT image group, as training data, and stores a model parameter 123a indicating the generated lesion identification model B1 in a model parameter storage unit 120. In addition, the training processing unit 132 generates a lesion identification model B2 (see FIG. 13) through machine learning using CT images included in the other CT image group, as training data, and stores a model parameter 123b indicating the generated lesion identification model B2 in the model parameter storage unit 120.

Based on the model parameters 123a and 123b, the lesion identification unit 143 calculates probability values as to being a lesion region, for each pixel, using the lesion identification models B1 and B2. The detection result output unit 145 integrates the calculated probability values at a ratio according to the image feature calculated by the image feature calculation unit 144 to output a final lesion detection result.

FIG. 13 is a diagram for explaining a lesion identification model generation process.

The image feature calculation unit 133 calculates an image feature of the same type as the image feature calculation unit 144 from each of the CT image sets 112 stored in the training data storage unit 110. Here, it is assumed that average luminance in the organ region is calculated as the image feature.

The training processing unit 132 classifies the CT image set 112 stored in the training data storage unit 110 into a first CT image group in which the image feature is included in the first range and a second CT image group in which the image feature is included in the second range. The training processing unit 132 generates the lesion identification model B1 through machine learning using the first CT image group as training data and generates the lesion identification model B2 through machine learning using the second CT image group as training data.

The first range regarding the image feature is assumed to be a range equal to or greater than a threshold value Nb1, and the second range is assumed to be a range equal to or less than a threshold value Nb2. In this case, substantially, the first CT image group corresponds to the CT image group with normal findings in the second embodiment, and the second CT image group corresponds to the CT image group with anomalous findings in the second embodiment. Accordingly, substantially, the lesion identification model B1 corresponds to normal findings, and the lesion identification model B2 corresponds to anomalous findings.

Here, the first range and the second range may be divided by one threshold value. However, in the present embodiment, the first and second ranges are set using the two threshold values Nb1 and Nb2, and Nb2 > Nb1 is satisfied. This ensures that the threshold values Nb1 and Nb2 are set such that a part of the first range on a lower limit side and a part of the second range on an upper limit side overlap with each other.

The range of the image feature (average luminance) that can be calculated from the CT image set with normal findings and the range of the image feature (average luminance) that can be calculated from the CT image set with anomalous findings are not consistently divided with a particular threshold value as a boundary. In a case where an image feature calculated from the CT image set has a value near the boundary between these ranges, it is not necessarily definite whether the CT image set has a normal finding or an anomalous finding. By overlapping a part of the first range and a part of the second range with Nb2 > Nb1 satisfied, the lesion identification models B1 and B2 can be generated in consideration of such indefiniteness. That is, even in a case where the feature of the CT image set input at the time of lesion detection has a value near the overlapping region between the first and second ranges, the lesion identification accuracy may be improved by using the lesion identification models B1 and B2.

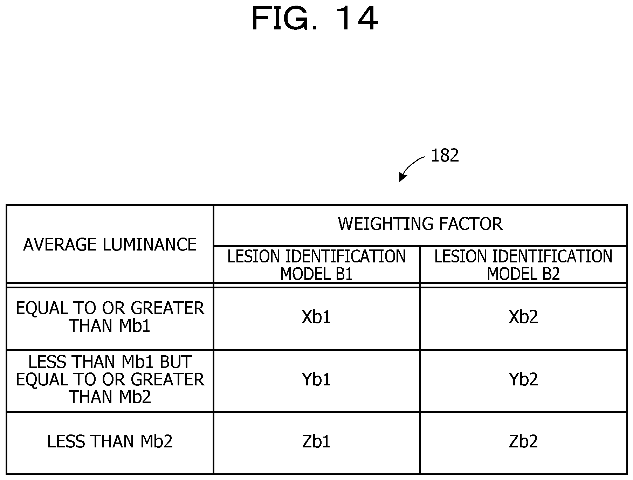

At the time of detecting a lesion region, it is sufficient to use the lesion identification models B1 and B2 instead of the lesion identification models A1 and A2 in FIG. 7, respectively, and to output a final lesion detection result by a procedure similar to that in FIG. 7. In addition, in the process of integrating the probability values from the lesion identification model B1 and the probability values from the lesion identification model B2, weighting factors as illustrated in FIG. 14 below can be simply used.

FIG. 14 is an example of a weight table illustrating a correspondence relationship between image features and weighting factors. The detection result output unit 145 can integrate the respective identification results of the lesion identification models B1 and B2 with reference to, for example, a weight table 182 as illustrated in FIG. 14.

In FIG. 14, as an example, a region that can be taken by the calculated average luminance is divided into three regions, using threshold values Mb1 and Mb2 (where Mb1 > Mb2 holds), and weighting factors for each of the lesion identification models B1 and B2 are associated with each of the divided regions. In a case where the average luminance is equal to or greater than the threshold value Mb1, Xb1 and Xb2 are applied as weighting factors corresponding to the lesion identification models B1 and B2, respectively. In a case where the average luminance is less than the threshold value Mb1 but equal to or greater than the threshold value Mb2, Yb1 and Yb2 are applied as weighting factors corresponding to the lesion identification models B1 and B2, respectively. In a case where the average luminance is less than the threshold value Mb2, Zb1 and Zb2 are applied as weighting factors corresponding to the lesion identification models B1 and B2, respectively.

Here, the weighting factors Xb1, Xb2, Yb1, Yb2, Zb1, and Zb2 each take a value equal to or greater than zero but equal to or less than one, and Xb1 + Xb2 = Yb1 + Yb2 = Zb1 + Zb2 = 1 holds. In a case where a weighting factor corresponding to one of the lesion identification models B1 and B2 has one, a weighting factor corresponding to the other has zero. In this case, the probability values output from the one lesion identification model will be used as it is.

In addition, it is estimated that more fat is accumulated as the average luminance is lower. Therefore, as the average luminance is lower, the weighting factors for the lesion identification model B2 corresponding to the anomalous findings are set to higher values. Specifically, Xb1 > Yb1 > Zb1 and Xb2 < Yb2 < Zb2 are satisfied. In addition, Xb1 > Xb2 and Zb1 < Zb2 are satisfied. The magnitude relationship between Yb1 and Yb2 only has to be determined according to the setting values of the threshold values Mb1 and Mb2.

By using such weighting factors, the detection result output unit 145 can increase the weight for the probability values by a lesion identification model having the condition of the CT images used as the training data closer to the condition of the input CT image group, among the lesion identification models B1 and B2, and can obtain the final lesion identification result. As a result, a lesion region may be accurately detected from the simple CT image.

Note that, the image features calculated by the image feature calculation units 133 and 144 may be, for example, a percentage of high luminance pixels having luminance equal to or greater than a predetermined threshold value in the organ region in the CT image group corresponding to one person, as well as the average luminance of the organ region as described above. In a case where such a percentage of high luminance pixels is used as the image feature, the lesion identification model B1 is generated using the CT image set in which the percentage of high luminance pixels is equal to or greater than a threshold value Nbla at the time of training. In addition, the lesion identification model B2 is generated using the CT image set in which the percentage of high luminance pixels is equal to or less than a threshold value Nb2a (> Nb1a). At the time of lesion detection, the weighting factors for the lesion identification model B2 only have to be set to higher values as the percentage of high luminance pixels is lower.

In addition, FIG. 13 illustrates an example in which two lesion identification models B1 and B2 are generated using two CT image sets having different ranges of the image feature. However, as another example, three or more lesion identification models may be generated using three or more CT image sets having different ranges of the image feature. In this case, at the time of lesion detection, the lesion identification results by each of the three or more lesion identification models can be simply integrated at a ratio according to the image feature.

In addition, a plurality of types of image features may be used. For example, the image feature calculation units 133 and 144 calculate the average luminance and the percentage of high luminance pixels described above. At the time of training, for example, Nbx is used as the threshold value of the average luminance, and Nby is used as the threshold value of the percentage of high luminance pixels. In this case, the CT image set 112 for training is classified into, for example, a CT image group having the average luminance ≥ Nbx and the percentage of high luminance pixels ≥ Nby, a CT image group having the average luminance ≥ Nbx and the percentage of high luminance pixels < Nby, a CT image group having the average luminance < Nbx and the percentage of high luminance pixels ≥ Nby, and a CT image group having the average luminance < Nbx and the percentage of high luminance pixels < Nby. Then, individual lesion identification models are generated using each of the classified CT image groups. At the time of lesion detection, the lesion identification results by each of the lesion identification models can be simply integrated at a ratio according to combinations of the average luminance and the percentage of high luminance pixels based on the input CT image set.

In addition, as in the second embodiment, calcification in the liver may be considered as an anomalous event that can change the relationship in luminance between the lesion region and the normal region. In this case, for example, the image feature calculation units 133 and 144 calculate the percentage of high luminance pixels described above. At the time of training, the lesion identification model B1 is generated using the CT image set in which the percentage of high luminance pixels is equal to or less than a threshold value Nb2b. In addition, the lesion identification model B2 is generated using the CT image set in which the percentage of high luminance pixels is equal to or greater than a threshold value Nblb (< Nb2a). At the time of lesion detection, the weighting factors for the lesion identification model B2 only have to be set to higher values as the percentage of high luminance pixels is higher.

Next, processes of the diagnosis support device 100a according to the third embodiment will be described with reference to flowcharts.

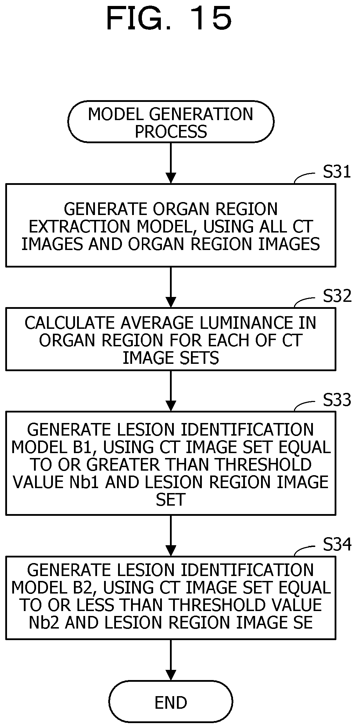

First, a procedure of a model generation process for generating the lesion identification models B1 and B2 described above will be described with reference to FIG. 15. FIG. 15 is an example of a flowchart illustrating a procedure of the model generation process.

[Step S31] The training processing unit 131 generates the organ region extraction model by performing machine learning using all the CT images 112a stored in the training data storage unit 110 and the organ region images 114a corresponding to each of the CT images 112a. In this machine learning, information on each pixel of the organ region images 114a is used as ground truth data. The training processing unit 131 stores a model parameter 121 indicating the generated organ region extraction model, in the model parameter storage unit 120.

[Step S32] The image feature calculation unit 133 calculates the average luminance in the organ region for each of the CT image sets 112 stored in the training data storage unit 110.

[Step S33] The training processing unit 132 extracts the CT image set 112 in which the calculated average luminance is equal to or greater than the threshold value Nb1, from among the CT image sets 112 stored in the training data storage unit 110. The training processing unit 132 extracts the CT image 112a including the organ region, from the extracted CT image set 112, based on the organ region image 114a.

The training processing unit 132 performs machine learning, using the extracted CT images 112a and lesion region images 113a corresponding to these CT image 112a, thereby generating the lesion identification model B1 corresponding to normal findings. In this machine learning, information on each pixel of the lesion region images 113a is used as ground truth data. The training processing unit 132 stores the model parameter 123a indicating the generated lesion identification model B1, in the model parameter storage unit 120.

[Step S34] The training processing unit 132 extracts the CT image set 112 in which the calculated average luminance is equal to or less than the threshold value Nb2, from among the CT image sets 112 stored in the training data storage unit 110. The training processing unit 132 extracts the CT image 112a including the organ region, from the extracted CT image set 112, based on the organ region image 114a.

The training processing unit 132 performs machine learning, using the extracted CT images 112a and the lesion region images 113a corresponding to these CT image 112a, thereby generating the lesion identification model B2 corresponding to anomalous findings. In this machine learning, information on each pixel of the lesion region images 113a is used as ground truth data. The training processing unit 132 stores the model parameter 123b indicating the generated lesion identification model B2, in the model parameter storage unit 120.

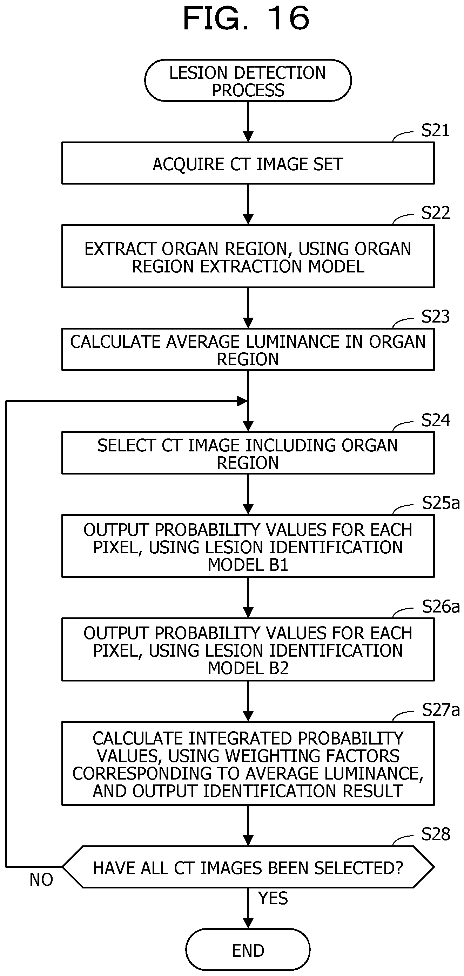

Next, a procedure of a lesion detection process for detecting a lesion region using the generated lesion identification models B1 and B2 will be described with reference to FIG. 16. FIG. 16 is an example of a flowchart illustrating a procedure of the lesion detection process.

In FIG. 16, processing steps having the same processing contents as those in FIG. 10 are indicated with the same step numbers denoted. In FIG. 16, steps S25a to S27a are executed instead of steps S25 to S27 in FIG. 10.

[Step S25a] The lesion identification unit 143 inputs the CT image to be processed to the lesion identification model B1 based on the model parameter 123a. This prompts the lesion identification unit 143 to output probability values as to being a lesion region, for each pixel of the CT image.

[Step S26a] The lesion identification unit 143 inputs the CT image to be processed to the lesion identification model B2 based on the model parameter 123b. This prompts the lesion identification unit 143 to output probability values as to being a lesion region, for each pixel of the CT image.

[Step S27a] The detection result output unit 145 acquires the weighting factors corresponding to the average luminance calculated in step S23 from the weight table 182. This ensures that the weighting factors corresponding to each of the lesion identification models B1 and B2 are acquired. The detection result output unit 145 multiplies each of the probability values output in steps S25a and S26a by the weighting factors corresponding to the lesion identification models B1 and B2 and adds up each of the obtained multiplication results, thereby calculating integrated probability values for each pixel. The detection result output unit 145 compares the calculated integrated probability value with a predetermined threshold value, identifies a pixel having an integrated probability value equal to or greater than the threshold value, as a lesion region, and identifies a pixel having an integrated probability value less than the threshold value, as a normal region. For example, the detection result output unit 145 generates and outputs a result image in which information indicating whether or not a pixel of the CT image is a lesion region is associated with each pixel.

### [Fourth Embodiment]

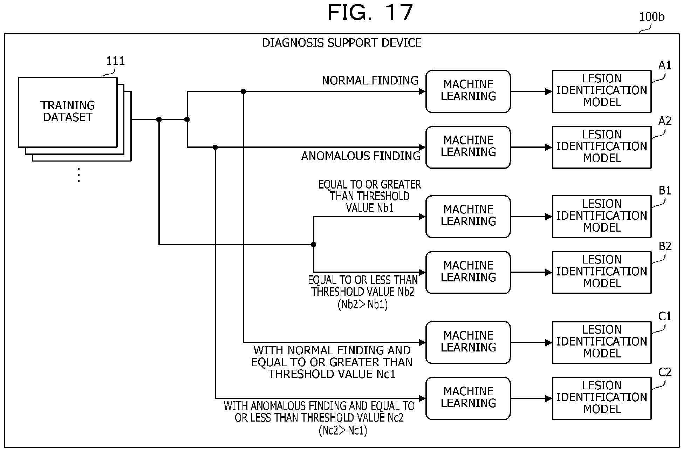

The lesion detection process may be executed using all the lesion identification models A1 and A2 generated in the second embodiment and the lesion identification models B1 and B2 generated in the third embodiment. Furthermore, lesion identification models C1 and C2 (see FIG. 17) generated in consideration of both of variations in findings and variations in image feature may be used. In the following fourth embodiment, an example of a diagnosis support device in which a lesion detection process using these lesion identification models A1, A2, B1, B2, C1, and C2 is executed will be described.

FIG. 17 is a diagram for explaining the model generation process of the diagnosis support device according to the fourth embodiment. The diagnosis support device 100b according to the fourth embodiment includes training processing units 131 and 132, an image feature calculation unit 133, an image acquisition unit 141, an organ region extraction unit 142, a lesion identification unit 143, an image feature calculation unit 144, and a detection result output unit 145 in FIG. 12.

The training processing unit 132 classifies the CT image set 112 included in a training dataset 111 into the CT image set 112 with normal findings and the CT image set 112 with anomalous findings by a procedure similar to that of the second embodiment. The training processing unit 132 generates a lesion identification model A1 through machine learning using the CT image set 112 with normal findings and generates a lesion identification model A2 through machine learning using the CT image set 112 with anomalous findings.

In addition, the training processing unit 132 classifies the CT image set 112 included in the training dataset 111 into the first CT image group in which the image feature is equal to or greater than a threshold value Nb1 and the second CT image group in which the image feature is equal to or less than a threshold value Nb2 (> Nb1) by a procedure similar to that of the third embodiment. The training processing unit 132 generates a lesion identification model B1 through machine learning using the first CT image group and generates a lesion identification model B2 through machine learning using the second CT image group.

Furthermore, the training processing unit 132 extracts a third CT image group made up of the CT image set 112 in which the image feature is equal to or greater than a threshold value Nc1, from the CT image set 112 with normal findings. In addition, the training processing unit 132 extracts a fourth CT image group made up of the CT image set 112 in which the image feature is equal to or less than a threshold value Nc2, from the CT image set 112 with anomalous findings. However, Nc2 > Nc1 holds. The training processing unit 132 generates a lesion identification model C1 through machine learning using the third CT image group and generates a lesion identification model C2 through machine learning using the fourth CT image group.

The lesion identification unit 143 calculates probability values as to being a lesion region, for each pixel, using the lesion identification models A1, A2, B1, B2, C1, and C2. The detection result output unit 145 integrates the calculated probability values at a ratio according to the image feature calculated by the image feature calculation unit 144 to output a final lesion detection result.

Here, in an actual CT image, a special case can occur in which a relationship between a finding and an image feature does not match a theoretical relationship, such as a case where an organ region appears relatively bright although fat is accumulated, for example. The lesion identification models C1 and C2 are trained models for enabling accurate detection of a lesion region even in a case where such a special case has occurred. For example, by setting the threshold value Nc1 to a relatively low value, the lesion identification model C1 can accurately identify a lesion region even in a case where a CT image set having the finding that fat is not accumulated but having relatively low average luminance is input. In addition, by setting the threshold value Nc2 to a relatively high value, the lesion identification model C2 can accurately identify a lesion region even in a case where a CT image set having the finding that fat is accumulated but having relatively high average luminance is input.

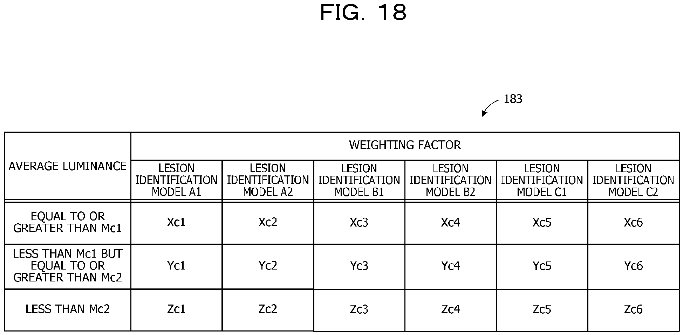

FIG. 18 is an example of a weight table illustrating a correspondence relationship between image features and weighting factors. The detection result output unit 145 can integrate the respective identification results of the lesion identification models A1, A2, B1, B2, C1, and C2 with reference to, for example, a weight table 183 as illustrated in FIG. 18.

In FIG. 18, as an example, a region that can be taken by the calculated average luminance is divided into three regions, using threshold values Mc1 and Mc2(where Mc1 > Mc2 holds). Then, weighting factors for each of the lesion identification models A1, A2, B1, B2, C1, and C2 are associated with each of the divided regions.

In a case where the average luminance is equal to or greater than the threshold value Mc1, Xc1 to Xc6 are applied as respective weighting factors corresponding to the lesion identification models A1, A2, B1, B2, C1, and C2. In a case where the average luminance is less than the threshold value Mc1 but equal to or greater than the threshold value Mc2, Yc1 to Yc6 are applied as respective weighting factors corresponding to the lesion identification models A1, A2, B1, B2, C1, and C2. In a case where the average luminance is less than the threshold value Mc2, weighting factors Zc1 to Zc6 are applied as respective weighting factors corresponding to the lesion identification models A1, A2, B1, B2, C1, and C2.

Here, each of the weighting factors Xc1 to Xc6, Yc1 to Yc6, and Zc1 to Zc6 takes a value equal to or greater than zero but equal to or less than one. Then, Xc1 + Xc2 + Xc3 + Xc4 + Xc5 + Xc6 = Yc1 + Yc2 + Yc3 + Yc4 + Yc5 + Yc6 = Zc1 + Zc2 + Zc3 + Zc4 + Zc5 + Zc6 = 1 holds.

Furthermore, as the average luminance is lower, the weighting factors for the lesion identification model A2 among the lesion identification models A1 and A2 is set to higher values, and the weighting factors for the lesion identification model B2 among the lesion identification models B1 and B2 is set to higher values. Specifically, Xc1 > Yc1 > Zc1, Xc2 < Yc2 < Zc2, Xc3 > Yc3 > Zc3, Xc4 < Yc4 < Zc4 are satisfied. In addition, Xc1 > Xc2, Zc1 < Zc2, Xc3 > Xc4, and Zc3 < Zc4 are satisfied. The magnitude relationship between Yc1 and Yc2 and the magnitude relationship between Yc3 and Yc4 only have to be determined according to the setting values of the threshold values Mc1 and Mc2.

Meanwhile, the lesion identification models C1 and C2 are generated through machine learning excluding training data of special cases such as a dark case even with a normal finding or a bright case even with fat accumulation (training data of outliers). Through such machine learning, more robust lesion identification models with normal findings and anomalous findings are generated. As for the weighting factors, Xc5 > Yc5 > Zc5, Xc6 < Yc6 < Zc6, Xc5 > Xc6, and Zc5 < Zc6 are satisfied. The magnitude relationship between Yc5 and Yc6 only has to be determined according to the setting values of the threshold values Mc1 and Mc2.

By using such weighting factors, the detection result output unit 145 can increase the weight for the probability values by a lesion identification model having the condition of the CT images used as the training data closer to the condition of the input CT image group, among the lesion identification models A1, A2, B1, B2, C1, and C2, and can obtain the final lesion identification result. As a result, a lesion region may be accurately detected from the simple CT image.

Next, processes of the diagnosis support device 100b according to the fourth embodiment will be described with reference to flowcharts.

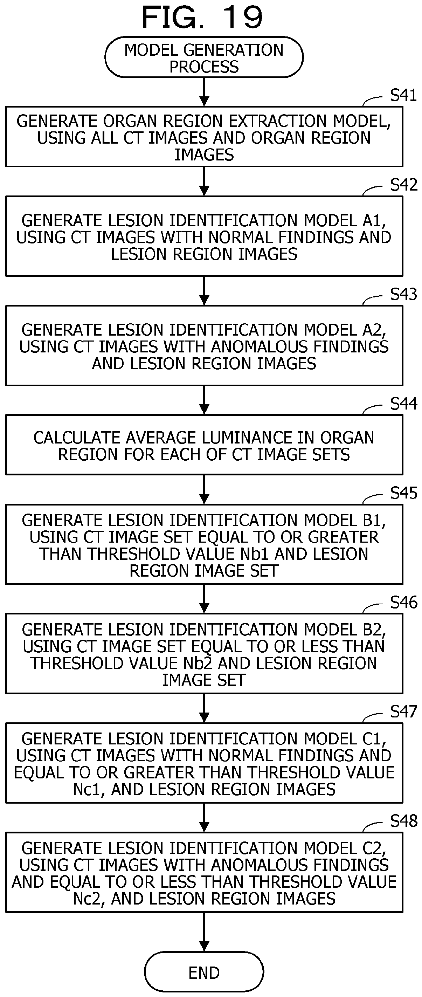

First, a procedure of a model generation process for generating the lesion identification models A1, A2, B1, B2, C1, and C2 described above will be described with reference to FIG. 19. FIG. 19 is an example of a flowchart illustrating a procedure of the model generation process.

[Step S41] The training processing unit 131 generates the organ region extraction model by a procedure similar to step S11 in FIG. 9 and step S31 in FIG. 15 and stores a model parameter 121 indicating the generated organ region extraction model, in a model parameter storage unit 120.

[Step S42] The training processing unit 131 generates the lesion identification model A1 by a procedure similar to step S12 in FIG. 9 and stores a model parameter 122a indicating the generated lesion identification model A1, in the model parameter storage unit 120.

[Step S43] The training processing unit 131 generates the lesion identification model A2 by a procedure similar to step S13 in FIG. 9 and stores a model parameter 122b indicating the generated lesion identification model A2, in the model parameter storage unit 120.

[Step S44] The image feature calculation unit 133 calculates the average luminance in the organ region for each of the CT image sets 112 stored in a training data storage unit 110 by a procedure similar to step S32 in FIG. 15.

[Step S45] The training processing unit 132 generates the lesion identification model B1 by a procedure similar to step S33 in FIG. 15 and stores a model parameter 123a indicating the generated lesion identification model B1, in the model parameter storage unit 120.

[Step S46] The training processing unit 132 generates the lesion identification model B2 by a procedure similar to step S34 in FIG. 15 and stores a model parameter 123b indicating the generated lesion identification model B2, in the model parameter storage unit 120.

[Step S47] The training processing unit 132 extracts the CT image 112a in which the average luminance calculated from the corresponding CT image set in step S44 is equal to or greater than the threshold value Nc1, from among the CT images 112a with normal findings used as the training data in step S42. The training processing unit 132 performs machine learning, using the extracted CT images 112a and lesion region images 113a corresponding to these CT image 112a, thereby generating the lesion identification model C1. In this machine learning, information on each pixel of the lesion region images 113a is used as ground truth data. The training processing unit 132 stores the model parameter indicating the generated lesion identification model C1, in the model parameter storage unit 120.

[Step S48] The training processing unit 132 extracts the CT image 112a in which the average luminance calculated from the corresponding CT image set in step S44 is equal to or less than the threshold value Nc2, from among the CT images 112a with anomalous findings used as the training data in step S43. The training processing unit 132 performs machine learning, using the extracted CT images 112a and the lesion region images 113a corresponding to these CT image 112a, thereby generating the lesion identification model C2. In this machine learning, information on each pixel of the lesion region images 113a is used as ground truth data. The training processing unit 132 stores the model parameter indicating the generated lesion identification model C2, in the model parameter storage unit 120.

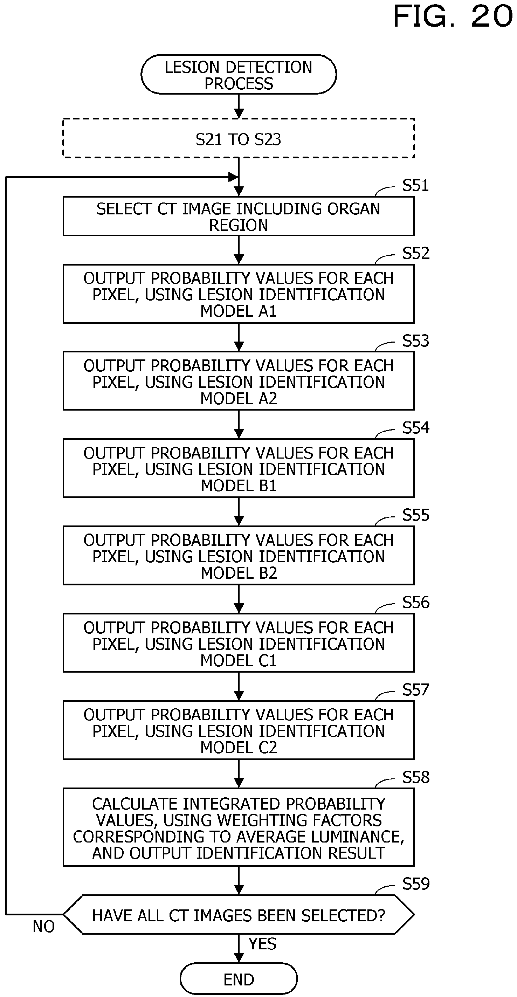

Next, a procedure of the lesion detection process for detecting a lesion region using the generated lesion identification models A1, A2, B1, B2, C1, and C2 will be described with reference to FIG. 20. FIG. 20 is an example of a flowchart illustrating a procedure of the lesion detection process.

In the process in FIG. 20, first, the processes in steps S21 to S23 in FIG. 10 are executed. This ensures that the average luminance in the organ region of the CT image set is calculated. Thereafter, the processes in step S51 and the subsequent steps are executed.

[Step S51] The lesion identification unit 143 selects the CT image to be processed, from among the CT images including the organ region.

[Step S52] The lesion identification unit 143 inputs the CT image to be processed to the lesion identification model A1 based on the model parameter 122a. This prompts the lesion identification unit 143 to output probability values as to being a lesion region, for each pixel of the CT image.

[Step S53] The lesion identification unit 143 inputs the CT image to be processed to the lesion identification model A2 based on the model parameter 122b. This prompts the lesion identification unit 143 to output probability values as to being a lesion region, for each pixel of the CT image.

[Step S54] The lesion identification unit 143 inputs the CT image to be processed to the lesion identification model B1 based on the model parameter 123a. This prompts the lesion identification unit 143 to output probability values as to being a lesion region, for each pixel of the CT image.

[Step S55] The lesion identification unit 143 inputs the CT image to be processed to the lesion identification model B2 based on the model parameter 123b. This prompts the lesion identification unit 143 to output probability values as to being a lesion region, for each pixel of the CT image.

[Step S56] The lesion identification unit 143 inputs the CT image to be processed to the lesion identification model C1 and outputs probability values as to being a lesion region, for each pixel of the CT image.

[Step S57] The lesion identification unit 143 inputs the CT image to be processed to the lesion identification model C2 and outputs probability values as to being a lesion region, for each pixel of the CT image.

[Step S58] The detection result output unit 145 acquires the weighting factors corresponding to the average luminance calculated in step S23 from the weight table 183. This ensures that the weighting factors corresponding to each of the lesion identification models A1, A2, B1, B2, C1, and C2 are acquired. The detection result output unit 145 multiplies each of the probability values output in steps S52 to S57 by the weighting factors corresponding to the lesion identification models A1, A2, B1, B2, C1, and C2 and adds up each of the obtained multiplication results, thereby calculating integrated probability values for each pixel. The detection result output unit 145 compares the calculated integrated probability value with a predetermined threshold value, identifies a pixel having an integrated probability value equal to or greater than the threshold value, as a lesion region, and identifies a pixel having an integrated probability value less than the threshold value, as a normal region. For example, the detection result output unit 145 generates and outputs a result image in which information indicating whether or not a pixel of the CT image is a lesion region is associated with each pixel.

[Step S59] The lesion identification unit 143 verifies whether all the CT images including the organ region have been selected. In a case where there is an unselected CT image, the process proceeds to step S51, and one unselected CT image is selected. On the other hand, in a case where all the applicable CT images have been selected, the lesion detection process ends.

Note that the diagnosis support device may execute the lesion detection process by combining two sets among the set of the lesion identification models A1 and A2, the set of the lesion identification models B1 and B2, and the set of the lesion identification models C1 and C2, for example.

For example, the lesion detection process is executed using the lesion identification models A1 and A2 and the lesion identification models C1 and C2. It can be said that the lesion identification models C1 and C2 are intended to cover even a special case in which a lesion region may not be accurately identified by the lesion identification models A1 and A2. Therefore, by using the lesion identification models A1 and A2 and the lesion identification models C1 and C2, a disadvantage of the lesion identification models A1 and A2 may be compensated, and the accuracy of lesion region identification may be enhanced.

In addition, the lesion detection process may be executed using the lesion identification models B1 and B2 and the lesion identification models C1 and C2. It can be said that the lesion identification models C1 and C2 are intended to cover even a special case in which a lesion region may not be accurately identified by the lesion identification models B1 and B2. Therefore, by using the lesion identification models B1 and B2 and the lesion identification models C1 and C2, a disadvantage of the lesion identification models B1 and B2 may be compensated, and the accuracy of lesion region identification may be enhanced.

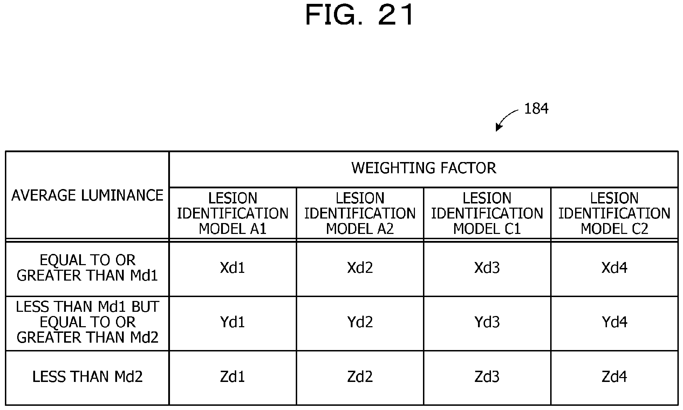

Here, as an example, weighting factors in a case where the lesion identification models A1 and A2 and the lesion identification models C1 and C2 are used are illustrated in FIG. 21.

FIG. 21 is an example of a weight table illustrating a correspondence relationship between image features and weighting factors. The detection result output unit 145 can integrate the respective identification results of the lesion identification models A1, A2, C1, and C2 with reference to, for example, a weight table 184 as illustrated in FIG. 21.

In FIG. 21, as an example, a region that can be taken by the calculated average luminance is divided into three regions, using threshold values Md1 and Md2 (where Md1 > Md2 holds). Then, weighting factors for each of the lesion identification models A1, A2, C1, and C2 are associated with each of the divided regions.

In a case where the average luminance is equal to or greater than the threshold value Md1, Xd1 to Xd4 are applied as respective weighting factors corresponding to the lesion identification models A1, A2, C1, and C2. In a case where the average luminance is less than the threshold value Md1 but equal to or greater than the threshold value Md2, Yd1 to Yd4 are applied as respective weighting factors corresponding to the lesion identification models A1, A2, C1, and C2. In a case where the average luminance is less than the threshold value Md2, Zd1 to Zd4 are applied as respective weighting factors corresponding to the lesion identification models A1, A2, C1, and C2.

Here, each of the weighting factors Xd1 to Xd4, Yd1 to Yd4, and Zd1 to Zd4 takes a value equal to or greater than zero but equal to or less than one. Then, Xd1 + Xd2 + Xd3 + Xd4 = Yd1 + Yd2 + Yd3 + Yd4 = Zd1 + Zd2 + Zd3 + Zd4 = 1 holds.

Furthermore, as the average luminance is lower, the weighting factors for the lesion identification model A2 among the lesion identification models A1 and A2 is set to higher values. Specifically, Xd1 > Yd1 > Zd1 and Xd2 < Yd2 < Zd2 are satisfied, and Xd1 > Xd2 and Zd1 < Zd2 are satisfied. The magnitude relationship between Yd1 and Yd2 only has to be determined according to the setting values of the threshold values Md1 and Md2.

Meanwhile, for the lesion identification models C1 and C2, as described above, the weighting factors when the average luminance has an intermediate value only have to be set to high values. Specifically, it is sufficient to satisfy Yd3 > Xd3 > Zd3 and Xd4 < Zd4 < Yd4. The magnitude relationship between Yd3 and Yd4 only has to be determined according to the setting values of the threshold values Md1 and Md2.

By using such weighting factors, the detection result output unit 145 can increase the weight for the probability values by a lesion identification model having the condition of the CT images used as the training data closer to the condition of the input CT image group, among the lesion identification models A1, A2, C1, and C2, and can obtain the final lesion identification result. As a result, a lesion region may be accurately detected from the simple CT image.

Note that the processing functions of the device (such as the information processing device 1 and the diagnosis support devices 100, 100a, and 100b) described in each of the above embodiments can be implemented by a computer. In that case, a program describing the processing contents of the functions supposed to be held by each device is provided, and the processing functions described above are implemented on the computer by execution of the program on the computer. The program describing the processing contents can be recorded on a computer-readable recording medium. Examples of the computer-readable recording medium include a magnetic storage device, an optical disc, and a semiconductor memory. Examples of the magnetic storage device include a hard disk drive device (HDD) and a magnetic tape. Examples of the optical disc include a compact disc (CD), a digital versatile disc (DVD), and a Blu-ray disc (BD, registered trademark).

In a case where the program is to be distributed, for example, portable recording media such as DVDs and CDs on which the program is recorded are sold. In addition, it is also possible to store the program in a storage device of a server computer and transfer the program to another computer from the server computer via a network.

The computer that executes the program stores, for example, the program recorded on the portable recording medium or the program transferred from the server computer in its own storage device. Then, the computer reads the program from its own storage device and executes a process in accordance with the program. Note that the computer can also read the program directly from the portable recording medium and execute a process in accordance with the program. In addition, the computer can also successively execute a process in accordance with the received program each time the program is transferred from the server computer coupled via the network.

All examples and conditional language provided herein are intended for the pedagogical purposes of aiding the reader in understanding the invention and the concepts contributed by the inventor to further the art, and are not to be construed as limitations to such specifically recited examples and conditions, nor does the organization of such examples in the specification relate to a showing of the superiority and inferiority of the invention. Although one or more embodiments of the present invention have been described in detail, it should be understood that the various changes, substitutions, and alterations could be made hereto without departing from the spirit and scope of the invention.

## Claims

1. A method for detecting lesion for a computer to execute a process comprising:
a training process that includes:
calculating a first image feature, based on a plurality of first tomographic image groups obtained by imaging an inside of each of a plurality of first human bodies, for each of the first tomographic image groups;
classifying tomographic images included in the plurality of first tomographic image groups into a plurality of second tomographic image groups, according to a range of the first image feature; and
generating a plurality of first lesion identification models configured to identify whether or not unit image regions included in the tomographic images to be identified are regions of a particular lesion, for each of the unit image regions, each through machine learning that uses different ones of the plurality of second tomographic image groups from each other, as training data; and
a lesion detection process that includes:
calculating a second image feature of a same type as the first image feature, based on a plurality of first tomographic images obtained by imaging the inside of a second human body;
acquiring, from each of the plurality of first lesion identification models, probabilities of being the regions of the particular lesion, for each of the unit image regions included in the plurality of first tomographic images, by inputting the plurality of first tomographic images to each of the plurality of first lesion identification models;
integrating, for each of the unit image regions included in the plurality of first tomographic images, the probabilities acquired from each of the plurality of first lesion identification models, based on the second image feature, to calculate an integrated value; and
detecting the regions of the particular lesion from each of the plurality of first tomographic images, based on the integrated value.

2. The method according to claim 1, wherein
the plurality of second tomographic image groups includes the second tomographic image groups that include the first tomographic image groups in which the first image feature is equal to or greater than a first threshold value, and the second tomographic image groups that include the first tomographic image groups in which the first image feature is equal to or less than a second threshold value lower than the first threshold value.

3. The method according to claim 1, wherein
the first image feature is calculated based on pixel information on the regions of a particular organ in image regions of the respective tomographic images included in the first tomographic image groups, and
the second image feature is calculated based on the pixel information on the regions of the particular organ in the image regions of the plurality of first tomographic images.

4. The method according to claim 3, wherein
the first image feature is a first average value that indicates an average of luminance in the regions of the particular organ in the image regions of the respective tomographic images included in the first tomographic image groups, and
the second image feature is a second average value that indicates the average of the luminance in the regions of the particular organ in the image regions of the plurality of first tomographic images.

5. The method according to claim 4, wherein
the calculating the integrated value includes calculating the integrated value by performing weighted addition on the probabilities acquired from each of the plurality of first lesion identification models, and
a higher weighting factor is set for the probabilities from the first lesion identification models generated by using the second tomographic image groups that have the lower first average value, among the plurality of first lesion identification models, as the second average value is lower.

6. The method according to claim 1, wherein
the training process includes:
classifying the plurality of first tomographic image groups into a plurality of third tomographic image groups that have different medical findings; and
generating a plurality of second lesion identification models configured to identify whether or not the unit image regions included in the tomographic images to be identified are the regions of the particular lesion, for each of the unit image regions, each through the machine learning that uses different ones of the plurality of third tomographic image groups from each other, as the training data,
the lesion detection process includes
acquiring the probabilities from each of the plurality of second lesion identification models, for each of the unit image regions included in the plurality of first tomographic images, by inputting the plurality of first tomographic images to each of the plurality of second lesion identification models, and
the calculating the integrated value includes
integrating, for each of the unit image regions included in the plurality of first tomographic images, the probabilities acquired from each of the plurality of first lesion identification models and each of the plurality of second lesion identification models, based on the second image feature, to calculate the integrated value.

7. The method according to claim 6, wherein
the classifying the plurality of first tomographic images into the plurality of third tomographic image groups includes classifying the plurality of first tomographic images into the third tomographic image groups that include the tomographic images with normal findings and the third tomographic image groups that include the tomographic images with anomalous findings, and
the training process includes:
generating a third lesion identification model configured to identify whether or not the unit image regions included in the tomographic images to be identified are the regions of the particular lesion, for each of the unit image regions, through the machine learning that uses, as the training data, the tomographic images of which the first image feature is included in a first range, among the tomographic images with the normal findings; and
generating a fourth lesion identification model configured to identify whether or not the unit image regions included in the tomographic images to be identified are the regions of the particular lesion, for each of the unit image regions, through the machine learning that uses, as the training data, the tomographic images of which the first image feature is included in a second range, among the tomographic images with the anomalous findings,
the lesion detection process includes
acquiring the probabilities from each of the third lesion identification model and the fourth lesion identification model, for each of the unit image regions included in the plurality of first tomographic images, by inputting the plurality of first tomographic images to the third lesion identification model and the fourth lesion identification model, and
the calculating the integrated value includes
integrating, for each of the unit image regions included in the plurality of first tomographic images, the probabilities acquired from each of the plurality of first lesion identification models, each of the plurality of second lesion identification models, the third lesion identification model, and the fourth lesion identification model, based on the second image feature, to calculate the integrated value.

8. A lesion detection program for causing a computer to execute a process comprising:
a training process that includes:
calculating a first image feature, based on a plurality of first tomographic image groups obtained by imaging an inside of each of a plurality of first human bodies, for each of the first tomographic image groups;
classifying tomographic images included in the plurality of first tomographic image groups into a plurality of second tomographic image groups, according to a range of the first image feature; and
generating a plurality of first lesion identification models configured to identify whether or not unit image regions included in the tomographic images to be identified are regions of a particular lesion, for each of the unit image regions, each through machine learning that uses different ones of the plurality of second tomographic image groups from each other, as training data; and
a lesion detection process that includes:
calculating a second image feature of a same type as the first image feature, based on a plurality of first tomographic images obtained by imaging the inside of a second human body;
acquiring, from each of the plurality of first lesion identification models, probabilities of being the regions of the particular lesion, for each of the unit image regions included in the plurality of first tomographic images, by inputting the plurality of first tomographic images to each of the plurality of first lesion identification models;
integrating, for each of the unit image regions included in the plurality of first tomographic images, the probabilities acquired from each of the plurality of first lesion identification models, based on the second image feature, to calculate an integrated value; and
detecting the regions of the particular lesion from each of the plurality of first tomographic images, based on the integrated value.
